Europäisches Patentamt

European Patent Office

Office européen des brevets

(11) Veröffentlichungsnummer: **0 304 934**
**A2**

(12) **EUROPÄISCHE PATENTANMELDUNG**

(21) Anmeldenummer: **88113957.0**

(22) Anmeldetag: **26.08.88**

(51) Int. Cl.4: **G01N 33/58 , G01N 33/535 , C07H 21/00 , //C12N15/00**

(30) Priorität: **26.08.87 DD 306365**
**26.08.87 DD 306366**
**26.08.87 DD 306367**

(43) Veröffentlichungstag der Anmeldung:
**01.03.89 Patentblatt 89/09**

(84) Benannte Vertragsstaaten:
**BE DE FR GB IT NL SE**

(71) Anmelder: **Akademie der Wissenschaften der DDR**
**Otto-Nuschke-Strasse 22/23**
**DDR-1086 Berlin(DD)**

(72) Erfinder: **Hunger, Hans-Dieter, Dr.rer.nat.**
**Zelterstrasse 84**
**DDR-1297 Zepernick(DD)**
Erfinder: **Behrendt, Gerhard, Dr.rer.nat.**
**Fritz-Reuter-Strasse 12**
**DDR-1603 Schulzerdorf(DD)**
Erfinder: **Schmidt, Gerhard, Dr.rer.nat.**
**Bielenweg 22**
**DDR-1115 Berlin(DD)**

(74) Vertreter: **Patentanwälte Beetz sen. - Beetz jun. Timpe - Siegfried - Schmitt-Fumian**
**Steinsdorfstrasse 10**
**D-8000 München 22(DE)**

(54) Markierte molekulare Sonden, Verfahren zu ihrer Herstellung und ihre Verwendung.

(57) Die Erfindung betrifft enzymmarkierte molekulare Sonden, Verfahren zu ihrer Herstellung und ihre Verwendung zum Nachweis von Biomolekülen durch eine Enzymreaktion. Das Verfahren zum Nachweis von Biomolekülen ist dadurch gekennzeichnet, daß in Lösung befindliche oder in Gelen enthaltene oder an eine feste Phase I gebundene Biomoleküle von einer an ein Enzym gekoppelten molekularen Sonde erkannt werden, das an die molekulare Sonde gekoppelte oder abgespaltene Enzym mit einem Substrat in Anwesenheit eines Cosubstrates zur Reaktion gebracht und das umgesetzte Substrat an einer festen Phase II oder III nachgewiesen wird. Insbesondere bezieht sich die Erfindung auf molekulare Sonden, die an Aminoglucosid-Phosphotransferasen gebunden sind. Die Erfindung ist vor allem in der Molekularbiologie, in der Genetik, Gentechnik, Medizin, Biologie, Landwirtschaft, Phytopathologie und im Veterinärwesen anwendbar. Mit dem erfindungsgemäßen Nachweisverfahren und bei deutlich verkürzten Auswerkzeiten wird ein wesentlich höherer Verstärkungsfaktor erzielt, wobei durch den Nachweis an einer zweiten festen Phase die Biomoleküle in mehreren gleiche oder verschiedenen Nachweisverfahren eingesetzt werden können.

## Markierte molekulare Sonden, Verfahren zu ihrer Herstellung und ihre Verwendung

Die Erfindung betrifft enzymmarkierte molekulare Sonden, Verfahren zu ihrer Herstellung und ihre Verwendung zum Nachweis von Biomolekülen durch eine Enzymreaktion. Anwendungsgebiete der Erfindung sind insbesondere die Molekularbiologie, Medizin, Biologie, Genetik, Phytopathologie, Gentechnologie und die Landwirtschaft sowie das Veterinärwesen. Nachweisverfahren von Biomolekuelen mit an Enzyme gekoppelten molekularen Sonden sind bekannt. Dabei wurden bisher sowohl immunreaktive Sonden (siehe z. B. Immunoenzymatic Techniques, Hrsg. S. Avrameas et al., Elsevier Science Publishers, 1983), Hybridisationssonden (siehe z. B. M. Renz, Chr. Kurz, Nucl. Acids Res. (1984) 12, S. 3 435 - 3 444) als auch Sonden mit spezifischer Affinitaet zu anderen Biomolekuelen an Enzyme gekoppelt. Als Kopplungsenzyme wurden vorwiegend alkalische Phosphatase, Galactosidase, Glucoamylase, Glucoseoxidase, Glucoronidase, Lactatdehydrogenase, Lactoperoxidase, Peroxidase, Ribonuclease und Tyrosinase mit ueblichen Vernetzungsreagenzien an molekulare Sonden gekoppelt (S. Avrameas, T. Ternynck, J. L. Guesdon, Scand. J. Immunol. (1978), S. 7 - 23). Bisher sind die in Nachweisverfahren eingesetzten Enzyme im wesentlichen auf die oben genannten beschraenkt, d. h. es ist nur eine begrenzte Anzahl von ihnen verwendet worden. Ein Grund fuer diese Beschraenkung ist die schlechte Nachweismoeglichkeit fuer andere Substrate. Es wurden bisher ueberwiegend Substrate verwendet, die nach der Enzymreaktion gefärbte Reaktionsprodukte ergeben. Bei Nachweisverfahren fuer Biomolekuele, die an feste Phasen gebunden sind, mittels enzymgekoppelter Sonden erfolgte der Nachweis des umgesetzten Substrates bisher stets auf der gleichen festen Phase, auf der sich auch das Biomolekuel befand, oder in Loesung. Der prinzipielle Ablauf eines solchen Nachweisverfahrens wird z. B. von R. H. Yolken in Immunoenzymatic Techniques, loc. cit, S. 274, fuer die Bestimmung eines Antigens (immunologischer Nachweis) erlaeutert:

1. Der gegen ein zu bestimmendes Antigen gerichtete Antikoerper 1 wird an der Wand einer Mikrotiterplatte fixiert;

2. Das Testmaterial wird zugegeben, vorhandenes Antigen wird durch den Antikoerper gebunden;

3. Nach Waschschritten wird ein enzymgekoppelter Antikoerper 2 zugegeben. Dieser reagiert mit dem an den Antikoerper 1 gebundenen Antigen;

4. Nach Waschschritten wird ein Substrat zugesetzt. Das an die Wand der Mikrotiterplatte fixierte Enzym (AK 1 - Antigen - AK 2 - Enzym) wandelt das Substrat in eine sichtbare Form um. Die in der Loesung messbare Farbintensitaet ist proportional zur Menge des Antigens im Testmaterial.

Bei diesen Nachweisreaktionen entstehen meist wasserloesliche Reaktionsprodukte. Wasserunloesliche Produkte werden auf den Traegermaterialien abgeschieden.

Durch die Eigenschaften der bisher verwendeten Enzyme bedingt, koennen an diese Sonden direkt gekoppelte Enzyme nicht unter Extrembedingungen eingesetzt werden (z. B. Hybridisationsbedingungen, bei hoher Temperatur oder in Gegenwart von Detergentien).

Bei Nachweisreaktionen von Biomolekuelen, bei denen das Biomolekuel auf einer festen Phase fixiert ist, wurde bisher der Nachweis immer auf der gleichen festen Phase durchgefuehrt, auf der sich das Zielmolekuel befindet (z. B. Nachweis eines Antigens nach Blotting aus einem Polyacrylamid-Gel auf Nitrocellulose mittels eines Peroxidase-gekoppelten Antikoerpers).

Bei diesem Nachweis wird das Antigen nach der Polyacrylamid-Gelelektrophorese zunaechst auf ein flaechiges Traegermaterial uebertragen und dort fixiert. Das Traegermaterial, auf dem sich das Antigen befindet, wird danach mit einem enzymgekoppelten Antikoerper inkubiert, der sich dabei an das Antigen bindet. Nach Waschschritten, in denen ueberschuessiger enzymgekoppelter Antikoerper ausgewaschen wird, wird ein loesliches Substrat, z. B. Diaminobenzidin oder 4-Chlornaphthol, in Anwesenheit von Wasserstoffperoxid zugesetzt. Die Peroxidase wandelt die Substrate in wasserunloesliche, farbige Verbindungen (Diaminobenzidin: braun, 4-Chlornaphthol: violett) um, die an den Stellen, an denen sich der Antigen-Antikoerper-Peroxidase-Komplex befindet, auf dem Traeger ausfallen und dadurch die Position eines Antigens innerhalb eines aufgetrennten Proteingemisches auf dem Traegermaterial markieren. Hierbei wird das Antigen auf dem gleichen Traegermaterial, auf dem es fixiert ist, farbig markiert. Eine Wiederverwendung solcher Blots nach der enzymatischen Nachweisreaktion mittels Farbstoffen ist schwer moeglich, da die gebundenen Farbstoffmolekuele meist Nebenreaktionen eingehen und damit zu Untergrundproblemen in Nachfolgeschritten fuehren.

Die Empfindlichkeit der Nachweisverfahren mittels Farbreaktionen ist stets geringer als die radioaktiver Nachweisverfahren. Prinzipiell aehnlich verlaeuft bisher der Nachweis von spezifischen Nucleinsaeuresequenzen mittels enzymgekoppelter Hybridisationssonden.

Insbesondere beim Nachweis von Nucleinsaeuresequenzen aus kleinsten Mengen biologischen Materials, z. B. zum Nachweis einzelner Gene aus minimalen DNS-Mengen, werden wesentlich empfindlichere Nachweismethoden benoetigt.

Weiterhin war es bisher nicht moeglich, Biomolekule durch enzymgekoppelte Sonden auf einer vom Biomolekuel unabhaengigen Traegerschicht nachzuweisen.

Als molekulare Sonden werden eine Vielzahl von chemischen Verbindungen und Biomolekuelen bezeichnet, die spezifische Affinitaet zu anderen Biomolekuelen aufweisen und deshalb zu deren Nachweis oder affinitaetschromatographischer Reinigung geeignet sind. Zu diesem Sonden zaehlen:
- immunreaktive Sonden, z. B. Antikoerper, Antiseren, immunologisch reaktive Proteine;
- Hybridisationssonden, z. B. Nucleinsaeurefragmente unterschiedlicher Kettenlaenge, DNS- und RNS-Molekuele;
- Proteine, die eine spezifische Affinitaet zu einem anderen Biomolekuel besitzen, z. B. Avidin Lectine, Streptavidin;
- organische Verbindungen, die eine spezifische Affinitaet zu Biomolekuelen besitzen, z. B. Biotin.

Der Nachweis spezifischer Biomolekuele (Zielmolekuele) erfolgt in der Molekularbiologie durch Reaktion der Zielmolekuele mit den molekularen Sonden (z. B. durch Immunreaktion, Hybridisierung), wobei die Sonden entweder direkt markiert (z. B. radioaktiv, Fluoreszenzfarbstoffe) oder mit einem zweiten Nachweissystem gekoppelt sind, das durch eine weitere, spezifische Reaktion zum Nachweis der Zielmolekuele fuehrt (z. B. Farbstoffabscheidung und anschliessende photographische Auswertung). Diese Nachweisverfahren sind in einer Vielzahl von Publikationen beschrieben und stellen den bekannten Stand der Technik dar. Innerhalb dieser Nachweisverfahren spielt die Kopplung von Enzymen an die Sonden eine zunehmende Rolle, siehe z. B. Immunoenzymatic Techniques, loc. cit. und Laboratory Techniques in Biochemistry and Molecular Biology, Vol. 15, Hrsg. R. H. Burdon und P. H. van Knippenberg, Kapitel 11, Preparation of Enzyme - Antibody or Other Enzyme Macromolecule Conjugates, S. 221 - 278, Amsterdam usw. (Elsevier Sciences Publishers) 1985.

Eine Reihe von Enzymen wurde z. B. an Antikoerper gekoppelt (S. Avrameas, T. Ternynck, J. L. Guesdon, loc. cit): alkalische Phosphatase, Galactosidase, Glucoamylase, Glucoseoxidase, Glucoronidase, Lactatdehydrogenase, Lactoperoxidase, Peroxidase, Ribonuclease, Tyrosinase. Nach Avrameas et al., loc. cit., muessen Enzyme, die zur Kopplung an molekulare Sonden verwendet werden sollen, bestimmte allgemeine Anforderungen erfuellen:

1. hohe spezifische Aktivitaet und Durchsatzrate des Substrates;

2. gute Stabilitaet bei Zimmertemperatur und bei Arbeitstemperatur;

3. kein oder geringer Aktivitaetsverlust nach der Kopplung;

4. leichte Zugaenglichkeit in hochreiner Form aus leicht zugaenglichen Materialien oder einfache Praeparation.

Diese Anforderungen werden von den bisher verwendeten Enzymen nur teilweise erfuellt. Die am meisten verwendeten Enzyme bei Nachweisreaktionen sind Peroxidase und alkalische Phosphatase. Die relativ geringe Thermostabilitaet der Enzyme bereitet dabei besonders beim Ansatz in Hybridisationsreaktionen (z. B. 48 Stunden bei 65 $^\circ$C) Schwierigkeiten bzw. macht einen Einsatz ganz unmoeglich.

Ein weiterer Nachteil der bisher verwendeten Enzyme besteht darin, dass zum Nachweis im allgemeinen Farbreaktionen genutzt werden, wobei nur eine relativ geringe Nachweisempfindlichkeit erreicht wird. Die Uebertragung von $^{32}$P-markiertem Phosphat wuerde einen wesentlich empfindlicheren Nachweis als bei den beschriebenen Verfahren erlauben. Die Uebertragung von $^{32}$P-markiertem Phosphat auf ein Substrat mittels eines an eine Sonde gekoppelten Enzyms wurde bisher jedoch nicht beschrieben.

Bei der Kopplung von Enzymen an molekulare Sonden werden als Kopplungsreagenzien in der Regel bifunktionelle Molekuele mit gleichen funktionellen Gruppen, z. B. Gutaraldehyd (siehe z. B. Avremeas, Bull. Soc. Chim. Biol. (1968)50, S. 1 169 ff), Benzochinon (siehe z. B. M. Renz, Ch. Kurz (1984), Nucl. Acids Res. 8, 3 435 - 3 444), Bisimidoester (siehe z. B. W. C. Mentzer et al., J. Protein Chem. (1982) 1, 141 - 155), oder mit unterschiedlichen Gruppen, z. B. Azidoaryloxysuccinimide (siehe z. B. T. H. Ji und J. Ji, Anal. Biochem. (1982) 121, S. 286 - 289), Azidoarylglyoxal (siehe z. B. S. M. Politz, H. F. Noller, P. D. McWirther, Biochemistry (1981) 20, 372-8) oder Azidoarylthiophthalimid (siehe z. B. R. B. Moreland et al., Anal. Biochem. (1982) 121, 321/6).

Zunehmende Bedeutung gewinnt das System Biotin-Avidin (Streptavidin), da durch seine Anwendung eine kaskadenartige Verstaerkung bis zum Faktor 8 erfolgen kann (siehe z. B. USP 4.463.090). Das Nachweissystem besteht meist in am Streptavidin oder Biotin gekoppelter Peroxidase oder alkalischer Phosphatase, so dass wieder eine Farbreaktion zum Nachweis genutzt wird. In den z. Z. empfindlichsten Hybridisationsverfahren (z. B. Nachweis einzelner Gene aus menschlicher DNS) sind diese nicht-radioaktiven Nachweisverfahren mit an Sonden gekoppelten Enzymen noch klar den Nachweisverfahren mit direkt radioaktiv markierten Nukleinsaeuresonden unterlegen. Speziell auf diesem Gebiet werden Methoden mit wesentlich hoeherer Nachweisempfindlichkeit benoetigt.

Mit den bisher bekannten Sonden lassen sich einerseits nur Verstaerkungen bis zum Faktor 8 unter Nutzung der Kaskade mit Biotin/Avidin erreichen, wobei aber an ein und denselben festen Traeger gearbeitet wird. Bei Nachweisverfahren unter Abscheidung eines Farbstoffes ist eine solche Verstaerkung nicht moeglich, ausserdem wird durch die niedergeschlagenen Farbstoffe in der Regel eine Wiederverwendung der Biomolekuele unmoeglich gemacht.

Der Nachweis mit enzymgekoppelten Sonden ist bisher an Faerbeverfahren gebunden, bei denen die hoehere Nachweisempfindlichkeit der autoradiographischen Methode nicht genutzt wird. Fuer einen radioaktiven Nachweis mit Verstaerkung des Signals stehen gegenwaertig keine Enzyme zur Verfuegung, die den Bedingungen aller Reaktionsschritte gewachsen sind.

Es ist Aufgabe der Erfindung, neuartige enzymmarkierte molekulare Sonden, Verfahren zu ihrer Herstellung und unter Verwendung dieser neuartigen enzymmarkierten molekularen Sonden ein Nachweisverfahren für Biomoleküle mit gesteigerter Empfindlichkeit zur Verfügung zu stellen. Dabei sollen die molekularen Sonden kopplungsfähige Enzyme aufweisen. Mit dem Verfahren zum Nachweis von Biomolekülen soll unter Verwendung dieser enzymmarkierten molekularen Sonden und einer speziellen festen Phase zur Bindung und zum Nachweis des umgesetzten Substrates eine höhere Nachweisempfindlichkeit erreicht werden.

Die Aufgabe wird anspruchsgemäß gelöst. Die abhängigen Ansprüche betreffen bevorzugte Ausführungsformen der Erfindungskonzeption.

Die erfindungsgemäßen enzymmarkierten molekularen Sonden bestehen aus einer molekularen Sonde und einem markierenden Enzym und sind dadurch gekennzeichnet, daß sie als Enzyme Transferasen (E.C.2) oder Ligasen (E.C.6) aufweisen, die direkt oder über einen Spacer an die jeweilige molekulare Sonde gekoppelt sind.

Besonders günstige, vorteilhafte Ausführungsformen der erfindungsgemäßen enzymmarkierten molekularen Sonden sind durch folgende alternative wie auch kumulative Merkmale gekennzeichnet:

Falls als molekulare Sonden Nucleinsäuren bzw. entsprechende Fragmente davon vorliegen, sind diese günstigerweise gentechnisch erzeugte Hybridisationssonden.

Geeignete Proteine sind Streptavidin, Avidin sowie kopplungsfähige Enzyme.

Bei den erfindungsgemäßen markierten molekularen Sonden ist es besonders vorteilhaft, wenn sie aus molekularen Sonden aufgebaut sind, die Affinität zu bestimmten Biomolekülen aufweisen. Die molekulare Sonde besteht nach einer günstigen Ausführungsform aus Biotin.

Eine geeignete Aminoglucosid-Phosphotransferase ist Neomycin-Phosphotransferase I oder II. In den Fällen, in denen die molekulare Sonde über einen Spacer an das Enzym gebunden ist, können die Bindungen zwischen der molekularen Sonde und dem Spacer wie auch die Bindungen zwischen dem Spacer und dem Enzym kovalent, ionisch und/oder über elektrostatische Wechselwirkungen vorliegen.

Die molekularen Sonden können vorteilhaft über Glutaraldehyd, Benzochinon-bks(succinimidylester), Bisimidate und/oder Azide an die Enzyme gekoppelt sein.

Die Spacer besitzen eine Kettenlänge von 1 bis 150 Atomen und können ggfs. eine oder mehrere Ringstrukturen aufweisen.

Günstig geeignete Spacermoleküle bzw. Spacergruppen sind solche, die photochemisch, reduktiv oder hydrolytisch spaltbar sind.

Die Kopplung zwischen molekularer Sonde und Enzym kann reversibel sein. Die molekulare Sonde und das Enzym können über Polyethylenimin gekoppelt sein.

Eine besondere Ausführungsform der erfindungsgemäßen markierten molekularen Sonden besteht darin, daß sie Fusionsproteine aus einem Protein und den Enzym darstellen.

Das Prinzip des erfindungsgemäßen Verfahrens zur Herstellung dieser enzymmarkierten molekularen Sonden besteht darin, daß an eine molekulare Sonde oder ein Gemisch molekularer Sonden direkt oder über einen Spacer eine Transferase (E.C.2) oder Ligase (E.C.6) gekoppelt wird.

Nach einer speziellen Ausführungsform des Verfahrens werden als enzymmarkierte molekulare Sonden Fusionsproteine, die aus einem Protein und einer Aminoglucosid-Phosphotransferase bestehen, hergestellt, besonders günstig nach einem gentechnischen Verfahren.

Besonders günstige, vorteilhafte Ausführungsformen des erfindungsgemäßen Verfahrens zur Herstellung der enzymmarkierten molekularen Sonden sind durch folgende Merkmale gekennzeichnet, die alternativ wie auch kumulativ vorliegen können:

Als Proteinkomponente kann beim erfindungsgemäßen Verfahren Protein A eingesetzt werden.

Als Nucleinsäuren eignen sich gentechnisch erzeugte Hybridisationssonden sowie Proteine mit spezifischer Affinität zu einem bestimmten Biomolekül, insbesondere Avidin, Streptavidin und kopplungsfähige Enzyme. Als molekulare Sonden können chemische Verbindungen mit Affinität zu einem bestimmten Biomolekül eingesetzt werden, besondes Bictin. Geeignete Aminoglucosid-Phosphotransferasen sind Neomycin-Phosphotransferase I und II.

Die Kopplung zwischen der molekularen Sonde und dem Enzym kann durch Glutaraldehyd, p-Benzochinon, Bis(succinimidylester), Bisimidate und/oder Azide vorgenommen werden.

Die Spacer können 1 bis 150 Atome lange Gruppierungen sein und eine oder mehrere Ringstrukturen aufweisen. Die Spacer können auch aus mehreren Einzelkomponenten aufgebaut sein.

Als Spacer eignen sich Polyethylenimine.

Das erfindungsgemäße Verfahren zum Nachweis von Biomolekülen durch markierte molekulare Sonden ist dadurch gekennzeichnet, daß in Lösung befindliche oder in Gelen enthaltene oder an eine feste Phase I gebundene Biomolekuele von einer an ein Enzym gekoppelten molekularen Sonde erkannt werden, das an die molekulare Sonde gekoppelte oder abgespaltene Enzym mit einem Substrat in Anwesenheit eines Cosubstrates zur Reaktion gebracht und das umgesetzte Substrat an einer festen Phase (I oder III nachgewiesen wird. Durch das erfindungsgemaesse Verfahren wird erstmalig eine Methode zur Verfuegung gestellt, mit der Erkennungsschritt und Nachweisschritt in Nachweisverfahren von Biomolekuelen unter Beteiligung von Enzymen an verschiedenen Phasen, z. B. zwei Flaechentraegern, durchgefuehrt werden und dadurch an der zweiten Phase die Verstaerkungsmoeglichkeiten durch die Enzymreaktion voll ausgeschoepft werden koennen.

Fuer das erfindungsgemaesse Verfahren sind nun eine ganze Reihe von Varianten moeglich, die jedoch alle in ähnlichen bzw. gleichen prinzipiellen Schritten ablaufen. Der Nachweis der Biomolekuele erfolgt erfindungsgemaess deshalb in folgenden Schritten:

a) Erkennungsschritt, in dem die durch ein gekoppeltes Enzym markierte molekulare Sonde an das in Loesung befindliche, im Gel enthaltene oder an eine feste Phase I gebundene Biomolekuel mit dafuer spezifischer Affinitaet angelagert wird;

b) Waschschritt, in dem ueberschuessige markierte molekulare Sonde ausgewaschen und/oder abgetrennt wird;

c) Enzymreaktionsschritt, in dem das Substrat in Anwesenheit eines Cosubstrates durch das Enzym umgesetzt wird;

d) Trennschritt, in dem nicht umgesetztes Cosubstrat und ggf. Substrat ausgewaschen oder abgetrennt wird;

e) Auswertungsschritt, in dem das umgesetzte Substrat an einer festen Phase II oder III gemessen oder durch geeignete Nachweisverfahren nachgewiesen wird.

Innerhalb eines jeden dieser Schritte sind auf Grund der vielfaeltigen Techniken zum Nachweis von Biomolekuelen wiederum eine Reihe von Variationen moeglich. So ist es z. B. denkbar, dass das nachzuweisende Biomolekuel in Loesung oder an einer festen Phase gebunden vorliegt. Dabei kann die feste Phase nicht nur ein teilchenfoermiger oder Flaechentraeger sein, sondern auch ein Gel, in dem die Biomolekuele frei beweglich sind, aber nicht gebunden vorliegen. Als Gele kommen die in der Molekularbiologie haeufig verwendeten Agarosegele und Polyacrylamid-Gele in Betracht, die in der Regel zu Trennoperationen verwendet werden.

Vielfältige Variationen sind in den Waschschritten und in den Trennschritten moeglich, da unter diesen eine Anzahl von Einzelschritten zusammengefasst sind, die nach bekannten Methoden durchgefuehrt werden. So koennen z. B. Waschungen an fester Phase mit verschiedenen, auf das System eingestellten Puffern durchgefuehrt werden. Es kann aber auch notwendig werden, eine oder mehrere Komponenten abzutrennen, was mittels Saeulen, Trenngelen oder durch eine chromatographische Methode erfolgen kann. Andererseits ist es moeglich, nur den Komplex von Biomolekuel und an das Enzym gekoppelter Sonde abzutrennen und dann im Enzymreaktionsschritt nachzuweisen. Es ist sogar moeglich, in einem dieser Schritte das Enzym wieder von der Sonde abzuspalten und dann nur das System auf Grund seiner Reaktion mit Substrat und Cosubstrat an einer festen Phase nachzuweisen.

Folglich existieren auch vielfaeltige Moeglichkeiten zur Ausgestaltung des Enzymreaktionsschrittes. Dieser kann ebenfalls in Loesung stattfinden, er kann an einer festen Phase oder in einem Gel durchgefuehrt werden. Wird der Enzymreaktionsschritt z. B. in Loesung durchgefuehrt, muss anschliessend das Umsetzungsprodukt des Substrates an eine feste Phase gebunden und dort nachgewiesen werden. Das bedeutet, dass ein Traegermittel zur Verfuegung gestellt wird, das das umgesetzte Substrat selektiv bindet. Wenn jedoch der Enzymreaktionsschritt bereits an fester Phase durchgefuehrt wird, d. h. das Substrat und/oder das Enzym, das noch an den Komplex aus Biomolekuel und molekularer Sonde gekoppelt oder bereits von ihm abgespalten ist, befinden sich an einem festen Traeger, dann wird das Substrat an der festen Phase umgesetzt und anschliessend, wobei nicht umgesetztes Cosubstrat sorgfaeltig abgetrennt werden muss, dort nachgewiesen. Auch wenn das Enzym, das von dem Komplex aus Biomolekuel und molekularer Sonde abgespalten worden ist, in Loesung vorliegt oder in einem Gel eingeschlossen ist, kann diese Umsetzung an einer festen Phase erfolgen und das umgesetzte Substrat dann an dieser nachgewiesen werden. Die Abtrennung des nicht umgesetzten Substrates kann dabei ebenfalls erfolgen, sie ist jedoch in den meisten Faellen nicht noetig.

Nachfolgend werden einige besonders günstige, vorteilhafte Ausführungsformen des erfindungsgemäßen Verfahrens zum Nachweis bzw. zur quantitativen Bestimmung von Biomolekülen schematisch aufgelistet:

Im Erkennungsschritt a) werden die an ein Enzym gekoppelte molekulare Sonde und das Biomolekül in Lösung aneinander angelagert;
im Waschschritt b) wird überschüssige markierte molekulare Sonde abgetrennt;
im Enzymreaktionsschritt c) wird das Enzym des Komplexes aus Biomolekül und markierter molekularer Sonde in Lösung mit dem Substrat, ggfs. in Gegenwart eines Cosubstrats, zur Reaktion gebracht;
im Trennschritt d) wird die Abtrennung des enzymatisch umgesetzten Substrats von überschüssigem Cosubstrat durch eine das Substrat selektiv bindende feste Phase II durchgeführt;
im Auswertungsschritt e) wird das umgesetzte Substrat an der festen Phase III gemessen oder autoradiographisch bestimmt.

Im Erkennungsschritt a) wird die durch ein gekoppeltes Enzym markierte molekulare Sonde an das an eine feste Phase I gebunden Biomolekül angelagert;
im Waschschritt b) wird überschüssige markierte molekulare Sonde ausgewaschen und/oder abgetrennt;
im Enzymreaktionsschritt c) wird das Enzym des an eine feste Phase I gebundenen Komplexes aus Biomolekül und markierter molekularer Sonde mit in Lösung befindlichem Substrat und Cosubstrat zur Reaktion gebracht;
im Trennschritt d) werden nicht umgesetztes Cosubstrat bzw. Substrat abgetrennt und umgesetztes Substrat an eine feste Phase III gebunden;
im Auswertungsschritt e) wird das umgesetzte Substrat an der festen Phase III gemessen oder autoradiographisch bestimmt.

Bei der vorgenannten Verfahrensweise kann die feste Phase I mit dem daran gebundenen Biomolekül und an diesem angelagerter markierter molekularer Sonde nach den Schritten a) und b) im Enzymreaktionsschritt c) mit einem Gel oder einem anderen Trenn- und Trägermaterial, in dem sich Substrat und Cosubstrat befinden, überschichtet werden, worauf im Trennschritt d) aus dem Gel oder dem Trenn- und Trägermaterial das umgesetzte Substrat auf eine feste Phase III übertragen und im Auswertungsschritt e) dort nachgewiesen werden kann.

Im Erkennungsschritt a) wird die durch ein gekoppeltes Enzym markierte molekulare Sonde an das an eine feste Phase I gebundene Biomolekül angelagert;
im Waschschritt b) wird überschüssige markierte molekulare Sonde ausgewaschen und/oder abgetrennt;
im Enzymreaktionsschritt c) wird das Enzym des an eine feste Phase I gebundenen Komplexes aus Biomolekül und markierter molekularer Sonde mit einem an eine feste Phase II gebundenen Substrat in Gegenwart eines sich in Lösung befindlichen Cosubstrats zur Reaktion gebracht;
im Trennschritt d) wird überschüssiges Cosubstrat ausgewaschen oder abgetrennt;
im Auswertungsschritt e) wird das umgesetzte Substrat an der festen Phase II gemessen oder autoradiographisch bestimmt.

Bei dieser Verfahrensweise kann im Erkennungsschritt a) das Biomolekül in einem oder mehreren Erkennungssystemen und in bzw. an einem oder mehreren Molekülen mit spezifischer Affinität erkannt oder mit einem oder mehreren Molekülen umgesetzt werden, worauf die mit einem Enzym gekoppelte molekulare Sonde mit mindestens einem dieser Moleküle zur Reaktion gebracht wird.

Die feste Phase I, die feste Phase II und/oder die feste Phase III können in Form eines Flachmaterials und/oder in Form eines optisch durchlässigen Materials eingesetzt werden.

Der Auswertungsschritt e) kann an optisch durchlässigen Materialen nach nicht-radioaktiven Verfahren mittels optischer Methoden durchgeführt werden.

Die Biomoleküle können auf die feste Phase I durch Typen oder nach Auftrennung in Gelen oder nach anderen Trenn-und Trägermaterialien durch Blotting-Verfahren aufgebracht werden.

Der Enzymreaktionsschritt a) kann mit dem an die molekulare Sonde gekoppelten Enzym oder mit dem von dieser abgespaltenem Enzym durchgeführt werden.

Geeignete Substrate beim erfindungsgemäßen Bestimmungsverfahren sind Chloramphenicol, Aminoglucosid-Antibiotika, Kanamycin, Neomycin, Gentamycin und Paromomycin, phosphorylierbare Proteine, besonders Histone, RNS, DNS, Desoxyribo- oder Ribooligonucleotide oder Desoxyribo- oder Ribopolynucleotide, doppelsträngige DNS, RNS, einsträngige DNS mit Starter (primer) und dgl.

Geeignete Cosubstrate sind $^{14}$C- oder $^{3}$H-markiertes Acetat.

Geeignete Cosubstrate sind ATP und/oder $\gamma$-$^{32}$-ATP.
Als Cosubstrate eignen sich Gemische der vier Desoxyribonucleosidtriphosphate, von denen eines oder mehrere in $\alpha$-Stellung $^{32}$P markiert sind. Als Cosubstrate können durch $^{35}$S, $^{3}$H oder $^{125}$J markiertes Desoxynucleosidtriphosphat oder Gemische von markierten oder ggfs. umarkierten Desoxynucleosid-triphosphaten eingesetzt werden. Biotinyliertes Desoxynucleosid-triphosphat stellt ein besonders geeignetes Cosubstrat dar. Ferner können auch als Cosubstrate DNS oder markierte DNS und/oder Desoxyribopolynucleotide eingesetzt werden.

Einige der bevorzugt durchgefuehrten Varianten werden im Folgenden naeher beschrieben.

Variante 1

a) Biomolekuele werden in Gelen oder anderen Trenn- und Traegermaterialien aufgetrennt und die die Biomolekuele enthaltenden Gele oder anderen Trenn- und Traegermaterialien werden mit einer Loesung, die eine an ein Enzym gekoppelte molekulare Sonde enthaelt, in Kontakt gebracht, wobei Biomolekuel und Sonde miteinander reagieren, so dass ein Komplex aus Biomolekuel und enzymgekoppelter molekularer Sonde im Gel entsteht (Erkennungsschritt);

b) im Waschschritt wird die ueberschuessige markierte molekulare Sonde aus dem Gel ausgewaschen oder abgetrennt;

c) im Enzymreaktionsschritt wird das Gel mit einem zweiten Gel oder Trenn- und Traegermaterial, das das Substrat und das Cosubstrat enthaelt, in Kontakt gebracht und das Substrat umgesetzt;

d) im Trennschritt wird das zweite Gel oder Trenn- und Traegermaterial mit dem zugesetzten Substrat mit einer festen Phase III in Kontakt gebracht und das umgesetzte Substrat selektiv an ihr gebunden;

e) nach Waschschritten wird im Nachweisschritt das umgesetzte Substrat auf der festen Phase III durch Messung der Radioaktivitaet, durch Autoradiographie oder ein anderes geeignetes Nachweisverfahren nachgewiesen.

In einer speziellen Ausfuehrungsform dieses Verfahrens wird nach den Schritten a und b das Gel, das den Komplex aus Biomolekuel und markierter molekularer Sonde enthaelt, mit einer festen Phase II, an die ein Substrat gebunden ist, in Gegenwart eines Cosubstrates in Kontakt gebracht und das enzymatisch umgesetzte Substrat an der festen Phase II nachgewiesen.

Diese spezielle Ausfuehrungsform laesst sich auch als Enzymtest zum Nachweis der Wirksamkeit des Enzymes verwenden, so dass dadurch eine weitere Anwendungsmoeglichkeit des erfindungsgemaessen Verfahrens gegeben ist.

Variante II

a) im Erkennungsschritt befinden sich die an ein Enzym gekoppelte molekulare Sonde und das Biomolekuel in Loesung und werden dort aneinander angelagert;

b) im Waschschritt wird ueberschuessige markierte molekulare Sonde abgetrennt;

c) im Enzymreaktionsschritt wird das Enzym des Komplexes aus Biomolekuel und markierter molekularer Sonde in Loesung mit dem Substrat in Gegenwart des Cosubstrates zur Reaktion gebraucht;

d) im Trennschritt wird die Abtrennung des enzymatisch umgesetzten Substrates vom ueberschuessigen Cosubstrat durch eine das Substrat selektiv bindende feste Phase III durchgefuehrt; und

e) im Auswertungsschritt wird das umgesetzte Substrat an der festern Phase III gemessen oder autoradiographisch bestimmt.

Der Schritt b), d. h. die Abtrennung der ueberschuessigen markierten molekularen Sonde, kann z. B. an Saeulen, insbesondere Affinitaetssaeulen, oder an Teilchensuspensionen mit einer Affinitaetswirkung erfolgen.

Der Enzymreaktionsschritt kann nach dieser Variante mit dem Enzym, das sich gekoppelt an der Sonde und diese wiederum angelagert an das Biomolekuel befindet, erfolgen, oder das Enzym kann durch einen zwischengeschalteten Schritt von dem Komplex aus Sonde und Bimolekuel abgespalten werden. Dabei kann diese Spaltung sowohl an der urspruenglichen Bindungsstelle von Enzym und molekularer Sonde erfolgen (d. h. es ist eine reversible Bindung) oder es kann in der Molekuelkette ("spacer"-Molekuel), die zur Kopplung von Enzym und molekularer Sonde verwendet worden ist, gespalten werden. Eine solche Spaltung ist natuerlich nicht nur in dieser Variante moeglich, sondern kann auch in anderen Varianten des erfindungsgemaessen Verfahrens durchgefuehrt werden.

## Variante III

a) Im Erkennungsschritt wird die durch ein gekoppeltes Enzym markierte molekulare Sonde an das an eine feste Phase I gebundene Biomolekuel angelagert;

b) im Waschschritt wird ueberschuessige markierte molekulare Sonde ausgewaschen und/oder abgetrennt;

c) im Enzymreaktionsschritt wird das Enzym das an eine feste Phase I gebundenen Komplexes aus Biomolekuel und markierter molekularer Sonde mit in Loesung befindlichem Substrat und Cosubstrat zur Reaktion gebracht;

d) im Trennschritt wird nicht umgesetztes Cosubstrat und ggf. Substrat abgetrennt und umgesetztes Substrat an eine feste Phase III gebunden; und

e) im Auswertungsschritt wird das umgesetzte Substrat an der festen Phase III gemessen oder autoradiographisch bestimmt.

Nach dieser Variante befinden sich sowohl das Biomolekuel, an das die molekulare Sonde angelagert wird, als auch das umgesetzte Substrat jeweils an einer festen Phase. Diese festen Phasen koennen gleich sein, sind dabei aber immer dem speziellen Biomolekuel und dem umgesetzten Substrat angepasst.

Der Enzymreaktionsschritt kann wieder mit dem vollstaendigen Komplex aus Biomolekuel, Sonde und gekoppelten Enzym oder mit dem abgespaltenem Enzym, wie es in der Variante II beschrieben worden ist, durchgefuehrt werden.

Eine weitere Variation dieser Verfahrensvariante besteht darin, dass die feste Phase I mit daran gebundenem Biomolekuel und daran angelagerter markierter molekularer Sonde nach den Schritten a) und b) mit einem Gel oder anderem Trenn- und Traegermaterial ueberschichtet wird, wobei sich darin Substrat und Cosubstrat befinden, so die Enzymreaktion durchgefuehrt und anschliessend aus dem Gel das umgesetzte Substrat auf eine feste Phase III uebertragen wird.

## Variante IV

Diese Variante stellt eine bevorzugte Ausfuehrungsform des erfindungsgemaessen Verfahrens dar. Die Verfahrensschritte werden danach wie folgt durchgefuehrt:

a) Im Erkennungsschritt wird die durch ein gekoppeltes Enzym markierte molekulare Sonde an das an eine feste Phase I gebundene Biomolekuel angelagert;

b) im Waschschritt wird ueberschuessige markierte molekulare Sonde ausgewaschen und/oder abgetrennt;

c) im Enzymreaktionsschritt wird das Enzym des an eine feste Phase I gebundenen Komplexes aus Biomolekuel und markierter molekularer Sonde mit einem an eine feste Phase II gebundenen Substrat in Gegenwart eines sich in Loesung befindlichen Cosubstrates zur Reaktion gebracht; und

d) im Trennschritt wird ueberschuessiges Cosubstrat ausgewaschen oder abgetrennt; und

e) im Auswertungsschritt wird das umgesetzte Substrat an der festen Phase II gemessen oder autoradiographisch bestimmt.

Bei dieser Variante besteht die Moeglichkeit, und das ist eine voellig neue Art der Durchfuehrung des Nachweises von Biomolekuelen, zwei feste Traeger, insbesondere Flaechentraeger, miteinander in Kontakt zu bringen und durch Zugabe der Loesung eines Cosubstrates die Enzymreaktion in Gang zu setzen. Dabei werden Flaechentraeger bevorzugt, z. B. solche, wie sie in DD-A C 07 G/296/754-2 beschrieben worden sind. Es ist jedoch ebenfalls moeglich, Teilchen miteinander umzusetzen, die unterschiedlich beschichtet sind, unter anderem auch Latexpartikel aus Kunststoffen mit einem magnetischen Kern.

Das Enzym kann im Reaktionsschritt c) sowohl an die molekulare Sonde gekoppelt als auch von ihr wieder abgespalten sein. Dieser letzte Fall ist besonders dann guenstig, wenn DNS als Substrat verwendet wird und das Enzym z. B. eine Ligase oder ein anderes Reparaturenzym ist.

Im Erkennungsschritt ist bei allen Verfahrensvarianten moeglich, dass bereit hier eine Verzweigung der Reaktion stattfindet oder dass zwischen das Biomolekuel und die markierte molekulare Sonde weitere Molekuele zwischengeschaltet werden. Das kann sich bei empfindlichen Enzymen als vorteilhaft erweisen. Es ist dabei moeglich, das bekannte Avidin/Biotin-System zu verwenden und damit eine moegliche Verstaerkung bereits im Erkennungsschritt auszunutzen. Als letzte Stufe des Erkennungsschrittes erkennt dabei die markierte molekulare Sonde nicht direkt das Biomolekuel selbst, sondern die vorhergehenden Reaktionspartner-Molekuele der Kaskade. Folglich besteht im Erkennungsschritt die Moeglichkeit, dass das Biomolekuel in einem oder mehreren Erkennungssystemen und einer oder mehreren Reaktionsstufen zunaechst von einem oder mehreren Molekuelen mit spezifischer Affinitaet erkannt bzw. mit einem oder mehreren Molekuelen eingesetzt wird und die mit einem Enzym gekoppelte molekulare Sonde mit mindestens einem dieser Molekuele reagiert.

Als feste Phasen sind eine Reihe von Materialien geeignet, die nach dem Stand der Technik bekannt sind und als Traegermaterialien fuer Biomolekuele eingesetzt werden. Die feste Phase I, an die das Biomolekuel gebunden ist, kann ein kovalent bindender, ionisch bindender oder ueber elektrostatische Wechselwirkungen bindender Traeger sein. Ueber elektrostatische Wechselwirkungen binden z. B. Nitrocellulose und Polyvinylidendifluorid, waehrend Polyamide (z. B. Xylen-Membranen) oder geladene Polyamide sowie Diethylaminoalkylcellulose ueber ionische Kraefte binden. Kovalent bindende Traeger sind z. B. derivatisierte Cellulose, z. B. Diazobenzyloxymethylcellulose, durch Cyanurchlorid akti vierte Cellulose (z. B. nach DD-WP 220.316). Darueber hinaus koennen Polystyrol ohne oder mit Aktivierung (z. B. durch Sulfonierung oder Aminierung/Diazotierung) oder chemisch aktiviertes Glas verwendet werden. Bevorzugt zur Durchfuehrung des erfindungsgemaessen Verfahrens werden feste Phasen I, die in Form von Flaechengebilden vorliegen.

Die feste Phase II ist ein Traeger, der das Substrat, das wiederum vom an die molekulare Sonde gekoppelten Enzym abhaengt, zu binden vermag. Auch hier koennen, in Abhaengigkeit vom Substrat, wieder kovalent bindende, ueber ionische Kraefte bindende oder ueber elektrostatische Wechselwirkungen bindende Traeger eingesetzt werden, wie sie bereits genannt worden sind. Folglich kommen fuer die feste Phase II wiederum Nitrocellulose, Polyvinylidendifluorid, Polyamid, geladenes Polyamid, Diethylaminoethylcellulose, durch Cyanurchlorid aktivierte Cellulose, aktiviertes Polystyrol, chemisch aktiviertes Glas oder andere derivatisierte Cellulosen in Betracht. Vor allem die ionisch bindenden Cellulosen, z. B. Phosphorcellulose (ein Umsetzungsprodukt der Cellulose mit Phosphorsaeure und daher Phosphorsaeuregruppen an den Anhydroglucoseeinheiten der Cellulose), Carboxymethylcellulose oder durch Cyanurchlorid und eine Verbindung, die eine Aminogruppe und eine Carbonsaeure-, Phosphorsaeure- oder Sulfonsaeuregruppe aufweist, modifizierte Cellulose, wie sie z. B. in der DD-A 296 754-2 oder in DD-A-237 844 beschrieben sind.

In einer besonderen Ausfuehrungsform kann die feste Phase II in form eines optisch durchlaessigen Mediums ausgebildet sein, z. B. einem Film, der das Substrat binden kann. Solche Materialien sind besonders zur routinemaessigen Auswertung, z. B. bei medizinischen Bestimmungsverfahren, besonders geeignet, da eine grosse Anzahl von Proben gleichzeitig maschinell ausgewertet werden kann. Die feste Phase II wird zum Nachweis bevorzugt als Flaechenmaterial eingesetzt; die Filme stellen dann den Fall eines optisch durchsichtigen Flaechenmaterials dar.

Die feste Phase III, die das Substrat und/oder das enzymatisch umgesetzte Substrat selektiv binden kann, kann nun ein Gel oder ein anderes Trenn- und Traegermaterial sein. Ebenso kann diese feste Phase III ein kovalent bindender, ein ionisch bindender oder ein ueber elektrostatische Wechselwirkungen bindender Traeger sein. Hier kommen, da die umgesetzten Substrate verschiedenartigster Natur sein koennen, im Prinzip die gleichen bekannten Materialien in Betracht, wie sie bereits weiter oben beschrieben worden sind, naemlich Nitrocellulose, Polyvinylidendifluorid, Polyamid, modifiziertes Polyamid, Diazobenzyloxymethylcellulose, Diethylaminoethylcellulose, durch Cyanurchlorid aktivierte Cellulose, aktiviertes Polystyren, chemisch aktiviertes Glas, Phosporcellulose, Carboxymethylcellulose oder durch Cyanurchlorid und eine Verbindung, die eine Aminogruppe und eine Carbonsaeure-, Phosphorsaeure- oder Sulfonsaeureguppe aufweist, modifizierte Cellulose. Auch die feste Phae III kann in Form eines optisch durchlaessigen Mediums verwendet werden. Es gelten hier die gleichen Moeglichkeiten und Vorteile wie bei der festen Phase II. Bevorzugt wird der Nachweis mit der festen Phase III als Flaechenmaterial durchgefuehrt, wenn sie z. B. als Film, Membran oder als Wirrfaservlies ausgebildet ist. Cellulosematerialien koennen in Papierform verwendet werden.

Die Auswertung bei den optisch durchlaessigen festen Phasen, insbesondere dann, wenn sie in Form eines Filmes ausgebildet sind, erfolgt mittels optischer Methoden, z. B. bei gefaerbten Produkten durch Densiometrie, durch Coloritmetrie oder Photographie. Die Biomolekuele koennen auf die feste Phase I nach ueblichen Verfahren aufgetragen werden. So werden sie insbesondere durch Tuepfeln, nach der Auftrennung in Gelen oder anderen Trenn- und Traegermaterialien durch sog. Uebertragungs- oder Transfertechniken (Blottingverfahren) aufgebracht.

Eine bevorzugte Ausfuehrungsform des erfindungsgemaessen Verfahrens besteht darin, dass Biomolekuele, die sich in Loesung befinden, im Gel enthalten oder an eine feste Phase I gebunden sein koennen, von einer Aminoglucosid Phosphotransferase-gekoppelten molekularen Sonde erkannt werden, die an die molekulare Sonde gekoppelte oder abgespaltene Aminoglucosid-Phosphotransferase mit dem Substrat in Gegenwart eines Cosubstrates zur Reaktion gebracht wird und das phosphorylierte Substrat an einer festen Phase II oder III nachgewiesen wird, wobei das Verfahren in folgenden wesentlichen Schritten durchgefuehrt wird:

a) Erkennungsschritt, in dem die durch eine gekoppelte Aminoglucosid-Phosphotransferase markierte molekulare Sonde an das in Loesung befindliche oder im Gel enthaltene oder an eine feste Phase I gebundene Biomolekuel mit dafuer spezifischer Affinitaet angelagert wird;

b) Waschschritt, in dem ueberschuessige markierte molekulare Sonde ausgewaschen und/oder abgetrennt wird;

c) Enzymreaktionsschritt, in dem die Phosphorylierung des Substrates durch Reaktion der Aminoglucosid-Phosphotransferase mit dem Substrat in Anwesenheit eines Cosubstrates durchgefuehrt wird;

d) Trennschritt, in dem nicht umgesetztes Cosubstrat und ggf. Substrat ausgewaschen oder abgetrennt wird;

e) Auswertungsschritt, in dem das phosphorylierte Substrat an einer festen Phase II oder III gemessen oder durch spezielle Nachweisverfahren nachgewiesen wird.

Aminoglucosid-Phosphotransferasen sind Enzyme, die unter Abspaltung eines Phosphatrestes vom Adenosintriphosphat (ATP) diesen Phosphatrest auf ein phosphorylierbares Substrat uebertragen. Das Substrat kann in Loesung vorliegen, in Traegern eingeschlossen sein oder an eine feste Phase gekoppelt sein. Das ATP wirkt bei dieser Reaktion als Cosubstrat.

Wenn an Stelle des ATP durch $^{32}$P markiertes ATP ($^{32}$P-gamma-ATP) verwendet wird, wird durch die Aminoglucosid-Phosphotransferase $^{32}$P-markiertes Phosphat auf das Substrat uebertragen. Das an die molekulare Sonde gekoppelte Enzym fuehrt diese Reaktion nicht nur einmal aus, sondern so lange wie folgende drei Bedingungen erfuellt sind;
- Vorhandensein von Substrat,
- Vorhandensein von Cosubstrat (d. h. ATP bzw. $^{32}$P-gamma-ATP),
- Sicherung der Bewegung des Enzyms an das Substrat.

Fuer das erfindungsgemaesse Verfahren ist jedoch wesentlich, dass jedes Enzymmolekuel mehrere Phosphatreste auf mehrere Substratmolekuele uebertraegt, so dass sich ein Verstaerkungsfaktor von mindestens 10 ergibt, der meistens jedoch wesentlich hoeher ist. Durch dieses Verfahren wird in der Phase, in der sich das Substrat befindet, ein verstaerktes Abbild der molekularen Sonde produziert. Nach dem erfindungsgemaessen Verfahren wird folglich der Nachweis nicht in der Phase gefuehrt, in der sich die molekulare Sonde befindet, sondern in der, in der sich das Substrat befindet. Indem bei einer molekularen Sonde mehrere Phasen mit Substrat in Gegenwart von Cosubstrat angeboten werden (beidseitig oder nacheinander), lassen sich von einem nachzuweisenden Biomolekuel mehrere verstaerkte Replikas herstellen. Vom urspruenglichen Bimolekuel kann der Komplex aus Sonde und Enzym nach ueblichen Verfahren wieder abgespalten werden und der Nachweis mit einer anderen, ggf. ebenfalls durch eine Aminoglucosid-Phosphotransferase markierte molekulare Sonde wiederholt werden.

Unter den Transferasen nehmen die Aminoglucosid-Phosphotransferasen eine Sonderstellung ein, wie ueberraschend gezeigt werden konnte:
- sie besitzen eine hohe Thermostabilitaet; das Enzym behaelt seine Aktivitaet auch nach Erwaermen auf 100°C, so dass lange Inkubationszeiten bei erhoehter Temperatur moeglich sind;
- sie weisen eine gute Detergentien -Stabilitaet auf; das Enzym ist trotz Veraenderung seiner Sekundaerstruktur durch Detergentien noch enzymatisch aktiv;
- ihre spezifische Aktivitaet ist sehr hoch;
- sie sind leicht zugaenglich und lassen sich aus Zellymaten von Mikroorganismen relativ leicht gewinnen.

Die Aminoglucosid-Phosphotransferasen erfuellen damit auf hervorragende Weise die Anforderungen an ein Enzym, das an andere Biomolekuele gekoppelt werden soll, wie sie von S. Avremeas in Bull. Soc. Chim. Biol. (1968) 50, S. 1 169 ff, genannt wurden. Sie koennen mikrobiologisch hergestellt werden, wie es z. B. von B. Reiss, R. Sprengel, H. Will und H. Schaller in Gene (1984) 30, S. 211 - 218 beschrieben worden ist.

Auf Grund der vielfaeltigen Moeglichkeiten, wie molekulare Sonden an die Aminoglucosid-Phosphotransferasen gekoppelt werden koennen, sind eine Reihe von Techniken zum Nachweis von Biomolekuelen moeglich. Zunaechst einmal sind als molekulare Sonden, die zur Kopplung geeignet sind, immunreaktive Sonden, Nucleinsaeuren, Proteine bzw. chemische Verbindungen mit Affinitaet zu einem Biomolekuel einsetzbar. Zu den immunreaktiven Sonden zaehlen monoklonale und polyklonale Antikoerper, Antiseren, Proteine, insbesondere Protein A, natuerlich vorkommende oder synthetisch hergestellte Peptide. Als Nucleinsaeuren koennen einstraengige, doppelstraengige oder partiell doppelstraengige DNS oder Fragmente davon, Oligodesoxyribonucleoside, RNS oder Fragmente davon oder Oligoribonucleotide verwendet werden, Proteine mit spezifischer Affinitaet zu einem Biomolekuel sind z. B. Avidin, Streptavidin, Pretamin, Gelatine, Fetain, Pepstatin, Lektine und viele Enzyme. Schliesslich sind chemische Verbindungen mit Affinitaet zu einem Biomolekuel Biotin, n-Aminophenylborsaeure und Cibacron Blue F3GA. Das Protein mit spezifischer Affinitaet zu einem anderen Biomolekuel ist dabei bevorzugt Avidin oder Streptavidin. Die chemische Verbindung mit Affinitaet zu einem Biomolekuel ist bevorzugt Biotin. Als Enzyme kommen zwei grosse Gruppen in Betracht, die Transferasen (nach E. C. Gruppe 2) und die Ligasen (E. C. Gruppe 6). Unter den Transferasen werden insbesondere die bevorzugt, die radioaktiv markierbare Gruppen uebertragen, z. B. Acyltransferasen (E.C. 2.3), Phosphor enthaltende Gruppen uebertragende Enzyme (E.C. 2.7) oder Polymerasen (E.C. 2.7.7). Insbesondere werden folgende Enzyme als besonders geeignet zur Durchfuehrung des erfindungsgemaessen Verfahrens betrachtet:
Proteinkinasen (E.C. 2.7.1),
Aminoglucosid-Phosphotransferasen (E.C. 2.7.1), insbesondere die Kanamycin-Phosphotransferase (E.C. 2.7.1.95),
T4-Polynucleotid-Kinase (E.C. 2.7.1.78)
Nucleotidyltransferasen (E.C. 2.7.7),
DNA-Polymerasen (E.C. 2.7.7.7),
Polynucleotid-Adenyltransferase (E.C. 2.7.7.19),
DNS-Nucleotidyloxotransferase (E.C. 2.7.7.31),
Revertase (E.C. 2.7.7.49),
Ligasen (E.C. 6),
Phosphorester bildende Enzyme (repair enzymes) (E.C. 6.5),
DNS-Ligasen (E.C. 6.9.11),
Jedes dieser Enzyme reagiert mit einem speziellen Substrat.

Die im folgenden aufgefuehrten Substrate gelten folglich nicht fuer jedes Enzym, sondern nur fuer solche, fuer die das Substrat spezifisch ist. Manche Substrate werden jedoch von mehreren Enzymen umgesetzt, jedoch in der Regel nach unterschiedlichen Mechanismen und auf unterschiedliche Art und Weise oder unter Bildung unterschiedlicher Reaktionsprodukte. Substrate sind also:
Chloramphenicol, Aminoglucosid-Antibiotika, insbesondere Kanamycin, Neomycin, Gentamycin und Paromomycin, phosphorylierbare Proteine, z. B. Histone;
DNS, RNS, Desoxyribo- oder Ribo-oligonucleotid, Desoxy- oder ribopolynucleotid, doppelstraengige DNS, einstraengige DNS plus Primer (Starter).

Die Enzyme benoetigen zusaetzlich zu ihrem Substrat ein Cosubstrat. Dieses Cosubstrat ist der Baustein, der zur Veraenderung des Substrates verwendet wird. Bei der Acyltransferase kann so das Cosubstrat z. B. ein $^{14}$C- oder $^3$H-markiertes Acetat sein. Bei den Phosphotransferasen ist das Cosubstrat in der Regel Adenosintriphosphat und/oder $^{32}$P markiertes Adenosintriphosphat (ATP). Daneben koennen auch die vier Desoxynucleosidtriphosphate (die des Adenosins, des Guanosins, des Cytosins und des Thymidins) uebertragen werden. Die Markierung der Desoxynucleosidtriphosphate kann in der alpha- oder in der gamma-Stellung erfolgen und je nach Enzym wird dann die eine oder andere Form umgesetzt . Ausser durch $^{32}$P koennen die Desoxynucleosidtriphosphate durch $^{35}$S, $^3$H oder $^{125}$I markiert sein, wobei jedes Nucleosid markiert sein kann, oder es kann nur ein Nucleosid markiert sein.

Weiterhin kann das Cosubstrat ein biotinyliertes Nucleotid sein, so dass nach der Enzymreaktion z. B. eine Reaktion mit an Avidin oder Streptavidin gebundenen Enzymen moeglich und damit eine Auswertung mit Farbstoffen genutzt wird.

Als Cosubstrate koennen darueber hinaus bereits markierte DNS oder Desoxyribopolynucleotide ggf. im Gemisch mit unmarkierten Molekuelen eingesetzt werden, die insbesondere als Cosubstrate fuer Ligasen in Betracht kommen.

Die bevorzugten Enzyme zur Durchfuehrung des erfindungsgemaessen Verfahrens sind die Aminoglucosid-Phosphotransferasen. Diese sind insbesondere die Aminoglucosid-Neomycin-Phosphotransferase I und II, die auch als Kanamycin-Phosphotransferasen oder APH 6 bzw. APH 3 bezeichnet werden. Substrate fuer die Aminoglucosid-Phosphotransferasen sind die Aminoglucosid-Antibiotika oder Aminocyclitol-Antibiotika (siehe dazu K. L. Rinehardt, J. Infect. Diseases (1969) 119, 345 - 50). Als solche koennen z. B. Neomycine, Kanamycine, Gentamycine und Tobramycin genannt werden. Bevorzugt wird als Substrat Kanamycin oder Neomycin. Das Cosubstrat ist markiertes oder nicht markiertes Adenosintriphosphat (ATP). Fuer den Nachweis wird ueblicherweise $^{32}$P-gamma-ATP verwendet, das jedoch mit nicht markiertem ATP vermischt sein kann.

Die bevorzugte Ausfuehrungsform des erfindungsgemaessen Verfahrens unter Verwendung einer Aminoglucosid-Phosphotransferase zum Nachweis der Biomolekuele besteht aus mehreren Verfahrensschritten, die wiederum unterschiedlich ausgefuehrt werden koennen, so dass eine Reihe von Ausfuehrungsformen fuer diese spezielle Gruppe von Enzymen existieren. Dabei haengt die jeweilige Ausfuehrungsform von der Art des Biomolekuels, von seiner Form, seiner Art der Bindung ggf. an einen Traeger, von der Art der Bindung des Substrates usw. ab.

Die nachzuweisenden Biomolekuele koennen in Loesung, in Gelen vor oder nach Trennoperationen, auf festen Traegern, durch Tuepfelverfahren auf feste Traeger uebertragen, nach Trennverfahren aus Gelen auf Flaechentraegern usw. vorliegen. Die Verfahren der Trennung, der Uebertragung und der Fixierung von Biomolekuelen auf Traeger sind dem Fachmann gut bekannt und werden deshalb hier nicht weiter erlaeutert. Nachdem die Biomolekuele in die nachzuweisende Form gebracht worden sind, werden folgende Schritte erfindungsgemaess durchgefuehrt;

a) Erkennungsschritt, in dem die durch eine gekoppelte Aminoglucosid-Phosphotransferase markierte molekulare Sonde an das Biomolekuel mit dafuer spezifischer Affinitaet angelagert wird;

b) Waschschritt, in dem ueberschuessige markierte molekulare Sonde ausgewaschen oder abgetrennt wird;

c) Enzymreaktionsschritt, in dem die an eine molekulare Sonde gekoppelte Aminoglucosid-Phosphotransferase mit dem Substrat unter Beteiligung eines Cosubstrates reagiert;

d) Trennschritt, in dem nicht umgesetztes Cosubstrat und ggf. Substrat ausgewaschen bzw. abgetrennt wird;

e) Auswertungsschritt, in dem das phosphorylierte Substrat gemessen oder durch spezielle Nachweisverfahren nachgewiesen wird.

Die Durchfuehrung eines jeden Schrittes ist an bestimmte Parameter gebunden, die kombinierbar sind und daher eine Vielzahl von Verfahrensvarianten zulassen. Innerhalb jeder Variante sind unterschiedliche Bedingungen fuer jeden Schritt moeglich, so dass spezielle Reaktionsbedingungen nicht allgemein, sondern nur fuer ein bestimmtes Beispiel angegeben werden koennen. Dazu sei auf die zur Erlaeuterung der Erfindung angefuegten Beispiele verwiesen.

Der Nachweis des phosphorylierten Substrates kann z. B. in Szintillationszaehlern oder durch Autoradiographie erfolgen. Die Autoradiographie wird besonders bei Flaechentraegern bevorzugt. Es sind jedoch auch andere Nachweisverfahren, z. B. Abtastverfahren oder Luminiszenzverfahren, moeglich. Ebenso wie fuer das erfindungsgemaesse Verfahren im allgemeinen sind fuer die bevorzugte Ausfuehrungsform Varianten in den einzelnen Schritten des Verfahrens moeglich. Einige dieser Varianten werden in den folgenden Ausfuehrungsvarianten beschrieben.

Ausfuehrungsvariante 1

Biomolekuele werden in Gelen oder anderen Trenn- und Traegermaterialien aufgetrennt.

a) Erkennungsschritt; die die Biomolekuele enthaltenden Trennmaterialien werden mit einer Loesung, die eine molekulare Sonde, an die eine Aminoglucosid-Phosphotransferase gekoppelt ist, in Kontakt gebracht, so dass Biomolekuel und Sonde aneinander angelagert werden und so ein Komplex aus Biomolekuel und enzymgekoppelter Sonde im Gel hergestellt wird;

b) Waschschritt: Auswaschen ueberschuessiger markierter molekularer Sonde aus dem Gel;

c) Enzymreaktionsschritt: das Gel, das den Komplex aus Biomolekuel und enzymgekoppelter Sonde enthaelt, wird mit einem zweiten Gel, das das Substrat und das Cosubstrat enthaelt, in Kontakt gebracht, so dass unter diesen Bedingungen das Substrat phosphoryliert wird;

d) das zweite Gel, das das phosphorylierte Substrat enthaelt, wird mit einer festen Phase III in Kontakt gebracht, und das phosphorylierte Substrat wird selektiv daran gebunden;

e) nach Waschschritten zur Entfernung ueberschuessigen Substrates und/oder Cosubstrates wird das phosphorylierte Substrat auf der festen Phase III durch Messung der Radioaktivitaet oder durch Autoradiographie nachgewiesen.

In einer weiteren Variante der Ausfuehrungsvariante 1 wird der Schritt c), der Enzymreaktionsschritt, so ausgefuehrt, dass das Gel, das den Komplex aus Biomolekuel und markierter molekularer Sonde enthaelt, mit einer festen Phase II, an die ein Substrat gebunden ist, in Gegenwart des Cosubstrates in Kontakt gebracht und das phosphorylierte Substrat auf der festen Phase II nachgewiesen wird.

Ausfuehrungsvariante 2

In dieser Variante befinden sich das Biomolekuel, ggf. in einem Gemisch mehrerer Biomolekuele, und die markierte molekulare Sonde in Loesung.

a) Erkennungsschritt: Eine molekulare Sonde, an die eine Aminoglucosid-Phosphotransferase gekoppelt ist, und das nachzuweisende Biomolekuel befinden sich in Loesung und werden in Loesung aneinander angelagert;

b) Waschschritt: ueberschuessige markierte molekulare Sonde wird abgetrennt;

c) Enzymreaktionsschritt: die Aminoglucosid-Phosphotransferase, die an die molekulare Sonde gekoppelt und dieser Komplex an das Biomolekuel angelagert ist, wird mit Substrat und Cosubstrat in Loesung umgesetzt;

d) Trennschritt: nicht umgesetztes Cosubstrat und ggf. Substrat werden abgetrennt;

e) Auswertungsschritt: die Aktivitaet des phosphorylierten Substrates wird gemessen.

Ausfuehrungsvariante 3

Diese Variante stellt eine weitere Moeglichkeit dar, wenn Biomolekuel und markierte molekulare Sonde in Loesung vorliegen, jedoch an einer festen Phase nachgewiesen werden sollen.

a) Erkennungsschritt: das nachzuweisende Biomolekuel und die molekulare Sonde, an die eine Aminoglucosid-Phosphotransferase gekoppelt ist, befinden sich in Loesung und werden dort miteinander umgesetzt;

b) Waschschritt: Abtrennung von ueberschuessiger markierter molekularer Sonde;

c) Enzymreaktionsschritt: die Aminoglucosid-Phosphotransferase, die an die molekulare Sonde gekoppelt und dieser Komplex an das Biomolekuel angelagert ist, wird in Loesung mit dem Substrat und dem Cosubstrat umgesetzt;

d) Trennschritt: durch einen das Substrat selektiv bindenden Traeger III erfolgt die Abtrennung des phosphorylierten Substrates vom ueberschuessigen Cosubstrat;

e) Auswertungsschritt: die Aktivitaet des phosphorylierten Substrates wird gemessen oder autoradiographisch bestimmt.

Ausfuehrungsvariante 4

Bei der Durchfuehrung dieser erfindungsgemaessen Verfahrensvariante befindet sich das Biomolekuel an eine feste Phase, z. B. einen Flaechentraeger oder eine Dispersion von Polymerteilchen, gebunden und reagiert nach der Anlagerung der markierten molekularen Sonde in Loesung mit Substrat und Cosubstrat.

a) Erkennungsschritt: an das an eine feste Phase I gebundene Biomolekuel wird die durch eine gekoppelte Aminglucosid-Phosphotransferase markierte molekulare Sonde angelagert;

b) Waschschritt: ueberschuessige markierte molekulare Sonde wird ausgewaschen oder abgetrennt;

c) Enzymreaktionsschritt: Reaktion des an eine feste Phase I gebundenen Komplexes aus Biomolekuel und markierter molekularer Sonde mit in Loesung befindlichem Substrat und Cosubstrat;

d) Trennschritt: nicht umgesetztes Cosubstrat und ggf. Substrat werden abgetrennt und phosphoryliertes Substrat ggf. an eine feste Phase III gebunden;

e) Auswertungsschritt: die Aktivitaet des phosphorylierten Substrates wird in Loesung oder ggf. an einer festen Phase III gemessen oder autoradiographisch bestimmt.

Diese Verfahrensvariante laesst sich durch Abaenderung des Enzymreaktionsschrittes weiter veraendern, indem dieser so durchgefuehrt wird, dass die feste Phase I mit daran gebundenem Biomolekuel und an diesem angelagerter markierter molekularer Sonde mit einem Gel ueberschichtet wird, wobei sich im Gel Substrat und Cosubstrat befinden, so die Enzymreaktion durchgefuehrt und anschliessend aus dem Gel das phosphorylierte Substrat auf eine feste Phase III uebertragen wird. Im Gel sind Substrat und Cosubstrat zwar in Loesung, aber gleichsam in "eingeschlossenem" Zustand (entrapped).

Ausfuehrungsvariante 5

Diese Variante stellt ein neuartiges Sandwich-Verfahren dar, in dem sowohl Sonde als auch Substrat an eine feste Phase gebunden sind und sich nur das Cosubstrat in Loesung befindet. Diese Anordnung ist besonders vorteilhaft, da durch die Verwendung von zwei festen Phasen die Bindung der molekularen Sonde an das Biomolekuel, die Bindung des Substrates und die Enzymreaktion unter ganz unterschiedlichen Reaktionsbedingungen durchgefuehrt werden koennen, die feste Phase mit gebundenem Komplex aus Bimolekuel und markierter molekularer Sonde unveraendert erhalten bleibt, deshalb mehrmals verwendet werden kann, oder die Sonde abgewaschen und ggf. durch andere ausgetauscht und so der Nachweis mit mehreren Sonden durchgefuehrt werden kann.

a) Erkennungsschritt: an eine feste Phase I ist ein Biomolekuel gebunden und an dieses wird eine durch eine Aminoglucosid-Phosphotransferase markierte molekulare Sonde angelagert;

b) Waschschritt: ueberschuessige markierte molekulare Sonde wird ausgewaschen und/oder abgetrennt;

c) Enzymreaktionsschritt: die Aminoglucosid-Phospotransferase des an die feste Phase I gebundenen Komplexes aus Biomolekuel und markierter molekularer Sonde wird mit einem an eine feste Phase II gebundenen Substrat in Gegenwart eines sich in Loesung befindlichen Cosubstrates zur Reaktion gebracht;

d) Trennschritt: ueberschuessiges Cosubstrat wird ausgewaschen oder abgetrennt;

e) Auswertungsschritt: die Aktivitaet des phosphorylierten Substrates an der festen Phase II wird gemessen oder au toradiographisch bestimmt.

In allen Ausfuehrungsvarianten der bevorzugten Ausfuehrungsform des erfindungsgemaessen Verfahrens ist es moeglich, dass im Erkennungsschritt die Reaktion von Biomolekuel und markierter molekularer Sonde nicht direkt, sondern ueber Zwischenstufen erfolgt. Das Biomolekuel kann z. B. zunaechst von einer Avidin- oder Streptavidin- bzw. Biotin-gekoppelten Sonde erkannt werden und diese danach mit einer Biotin-bzw. Avidin- oder Streptavidin-gekoppelten Aminoglucosid-Phosphotransferase umgesetzt werden. Dieses Prinzip laesst sich verallgemeinern, dahingehend, dass im Erkennungsschritt das Biomolekuel in einem oder mehreren Erkennungssystemen und in einer oder mehreren Reaktionsstufen zunaechst von einem oder mehreren Molekuelen mit spezifischer Affinitaet erkannt oder mit einem oder mehreren Molekuelen umgesetzt wird und die mit einer Aminoglucosid-Phosphotransferase gekoppelte molekulare Sonde mit mindestens einem dieser Molekule reagiert. Damit wird eine weitere Verstaerkung erreicht, zumal das Verfahren einmal oder mehrmals wiederholt werden kann.

Schliesslich ist es ebenfalls moeglich, den Enzymreaktionsschritt nach dem Erkennungs- und Waschschritt so durchzufuehren, dass der Komplex aus Biomolekuel und markierter molekularer Sonde an der Kopplungsstelle aus Aminoglucosid-Phosphotransferase und molekularer Sonde wieder gespalten wird, d. h. dass die Kopplung mit Reagenzien erfolgt, die entweder selbst spaltbar sind oder dass die Kopplung an mindestens einem der beiden Reaktionspartner reversibel ist. Das Enzym kann dann in Loesung oder nach Bindung an eine andere feste Phase in Gegenwart des Cosubstrates das in Loesung befindliche oder an eine feste Phase gebundene Substrat phosphorylieren.

Bei der bevorzugten Ausfuehrungsform des erfindungsgemaessen Verfahrens koennen die zur Bindung der Biomolekuele, Sonden oder Substrate verwendeten Traeger wiederum in verschiedener Form vorliegen, z. B. als Pulver, Granulate, Gele, Kugeln, Fasern und als Dispersion, Emulsion, Latex, Film, Saeulenfuellung, Matrix, Vlies oder allgemein als Flaechentraeger ausgebildet sein. Das Material koennen natuerliche oder kuenstliche Polymere oder deren Gemische sein. Die Bindung erfolgt ueber kovalente Bindung, wenn die feste Phase durch entsprechende chemische Verbindungen, z. B. Cyanurchlorid oder Bromcyan, aktiviert wurde. Eine Bindung ist auch ueber ionische Bindungen oder elektrostatische Wechselwirkungen moeglich.

Als feste Phase I sind deshalb Traegermaterialien geeignet, die nach allen drei Bindungsprinzipien zu binden vermoegen. Beispiele fuer feste Phasen, die ueber elektrostatische, d. h. z. B. ueber hydrophobe, Dipol-Dipol- oder van der Waals`sche Kraefte, Wechselwirkungen binden, sind u. a. Nitrocellulose oder Polyvinylidendifluorid. Beispiele fuer feste Phasen, die ueber ionische Kraefte binden, sind z. B. modifizierte, ionisch geladene Polyamide, insbesondere Polyamidmembranen (Nylonmembranen), durch ionische Gruppen substituierte Cellulose, wie Diethylaminoethylcellulose, und sulfoniertes Polystyrol. Beispiele fuer kovalent bindende feste Phasen sind Diazobenzyloxymethylcellulose, durch Cyanurchlorid aktivierte Cellulose, durch Bromcyan aktivierte Cellulose oder aktiviertes Polystyrol oder Glas. Verwendbar ist weiterhin chemisch aktiviertes Glas, das ueber ionische und/oder kovalente Bindungen zu binden vermag. Besonders bevorzugt zur Durchfuehrung des erfindungsgemaessen Verfahrens werden Flaechentraeger aus den oben beschriebenen Materialien.

Die feste Phase II ist vor allem ein ionisch bindender Traeger mit negativen Ladungen oder ein kovalent bindender Traeger. Als negativ ionisch geladene Traeger werden vor allem Cellulosederivate bevorzugt, z. B. Phosphorcellulose, Carboxymethylcellulose, durch Cyanurchlorid modifizierte Cellulose, die anschliessend mit einer Verbindung, die mindestens eine Aminogruppe und eine Carbonsaeure-, Phosphorsaeure- oder Sulfonsaeuregruppe im Molekuel aufweist, umgesetzt wird, wie in DD-A 237 844 beschrieben ist, worauf hier bezug genommen wird. Als kovalent bindende Traeger sind die gleichen, wie sie fuer die feste Phase I benannt wurden, geeignet. Besonders bevorzugt fuer das erfindungsgemaesse Verfahren wird die feste Phase II in Form von Flaechenmaterialien.

Die selektiv bindende feste Phase III ist ein negativ ionisch geladener Traeger. Hier sind wiederum Cellulosederivate oder sulfonierte Polystyrole oder durch Acrylate mit Carbonsaeuregruppen beschichtete Materialien geeignet. Bevorzugt werden Phosphorcellulose, Carboxymethylcellulose oder Cellulosederivate nach DD-WP 237.844, die aus Cellulose durch Aktivierung mit Cyanurchlorid und anschliessender Umsetzung mit einer Verbindung, die mindestens eine Aminogruppe und mindestens eine Carbonsaeure-, Phosphorsaeure- oder Sulfonsaeuregruppe im Molekuel enthaelt, hergestellt werden.

Ein bevorzugtes Verfahren zur Kopplung des Substrates an feste Phasen ist in DD-A 254 012 beschrieben, worauf hier bezug genommen wird. Die Kopplung des Enzyms an die molekulare Sonde zu ihrer Markierung kann nach verschiedenen Verfahren, die fuer bestimmte Enzyme beschrieben sind, erfolgen. Die Bindung der Sonde an das Enzym kann z. B. kovalent durch eine Reihe von bifunktionellen Kopplungsreagenzien erfolgen. Derartige Kopplungsreagenzien sind z. B. Glutaraldehyd, Benzochinon, Bis-succinimidylester wie Bis-sulfosuccinimidylsuberat und Disuccinimidyltartrat, Bisimidate wie Dimethylsuberimidat und Dimethyladipimidat, Azide wie N-5-Azido-2-nitrobenzoyloxysuccinimid oder 4,4´-Dithio-bis(phenylazid). Dabei kann ein Kopplungsmolekuel zwei unterschiedliche Gruppen tragen oder ggf. im Molekuel reduktiv oder photochemisch spaltbare Gruppen aufweisen. Beispiele fuer solche Kopplungsreagenzien sind N-Succinimidyl-(4-azidophenyl)-1,3´-dithiopropionat oder Sulfosuccinimidyl-2-(m-azido-o-nitrobenzamido)-ethyl-1,3´-dithiopropionat.

Weiterhin koennen molekulare Sonde und Enzym direkt kovalent miteinander verbunden sein. Eine solche Kopplung kann z. B. in Gegenwart von Kondensationsmitteln wie Carbodiimiden durchgefuehrt werden.

Die Bindung des Enzyms an die molekulare Sonde kann ebenso ueber ionische Bindungen erfolgen. Eine ionische Bindung kann z. B. direkt zwischen Protein und DNS gebildet werden. In der Regel werden jedoch Reagenzien zugesetzt, die eine Bindung ermoeglichen, z. B. Polyethylenimine unterschiedlicher Molmassen, Polyelektrolyte oder Ampholyte.

Da Enzyme ueber Hydrophobe und hydrophile Bereiche verfuegen, ist es moeglich, sie auch ueber elektrostatische Wechselwirkungen an die molekulare Sonde zu binden. Als elektrostatische Wechselwirkungen sollen weiterhin van der Waals'sche Kraefte, Dipol-Dipol-Wechselwirkungen und Wasserstoffbrueckenbindungen verstanden werden.

Ein besonderer Fall ist es, wenn ein Fusionsprotein als markierte molekulare Sonde verwendet wird, das entweder natuerlich vorkommt oder gentechnisch erzeugt werden kann. Bei diesem sind Enzym und molekulare Sonde bereits miteinander verbunden.

Die Kopplungsstelle von molekularer Sonde und Enzym kann spaltbar sein (z. B. enzymatisch).

Die Kopplung der fuer die bevorzugte Ausfuehrungsform des erfindungssgemaessen Verfahrens verwendeten Aminoglucosid-Phosphotransferasen an die molekularen Sonden ist neu und damit sind auch die so hergestellten enzymmarkierten molekularen Sonden neue Produkte; sie stellen einen weiteren Gegenstand des erfindungsgemaessen Verfahrens dar. Die Herstellung dieser durch Aminoglucosid-Phosphotransferasen markierten molekularen Sonden ist ebenfalls neu und ist ein weiterer Gegenstand des erfindungsgemaessen Verfahrens. Erfindungsgemaess werden die molekularen Sonden an die Aminoglucosid-Phosphotransferasen direkt oder ueber einen Spacer gekoppelt.

Es wurde ueberraschend gefunden, dass Aminoglucosid-Phosphotransferasen Eigenschaften aufweisen, die sie zur Verwendung beim Nachweis von Biomolekuelen unter Signalverstaerkung besonders geeignet machen. Diese besonderen Eigenschaften sind:
- eine hohe Thermostabilitaet: die Enzyme behalten auch nach Erwaermen auf 100° C ihre Aktivitaet, erfuellen die Anforderungen an Lagerstabilitaet und gewaehrleisten lange Inkubationszeiten sowohl waehrend Erkennungsreaktionen vom Biomolekuel und Sonde als auch waehrend der Reaktion des Enzyms mit dem Substrat;
- Detergenzien-Stabilitaet: diese Enzyme sind im Gegensatz zu anderen Enzymen trotz Veraenderung ihrer Sekundaerstruktur durch Detergenzien noch enzymatisch aktiv;
- hohe spezifische Aktivitaet: durch Uebertragung von $^{32}P$-markiertem Phosphat auf ein Substrat lassen sich radioaktiv hochmarkierte Substrate herstellen;
- leichte Zugaenglichkeit: die Aminoglucosid-Phosphotransferasen koennen relativ leicht aus Zellysaten von Mikroorganismen gewonnen werden.

Die Faehigkeit der Aminoglucosid-Phosphotransferasen, ohne Schaedigung auch bei fuer Enzyme harten Reaktionsbedingungen radioaktiv markiertes Phosphat zu uebertragen und ihre Arbeitsfaehigkeit nach den ueblichen Hybridisationsbedingungen (d. h. bis zu 48 Stunden bei 65° C) zeigen deutlich die Sonderstellung dieser Enzyme und machen sie deshalb fuer Nachweisverfahren besonders geeignet.

Durch die erfindungsgemaessen, markierten molekularen Sonden werden Reaktionsprodukte erzeugt, die hoch radioaktiv und damit leicht nachweisbar sind. Der Nachweis gelingt auch noch bei Konzentrationen der Zielmolekuele, die nach den bekannten Nachweisverfahren nicht nachweisbar sind.

Geeignete molekulare Sonden zur Kopplung mit den Aminoglucosid-Phosphotransferasen sind:

a) immunreaktive Sonden, insbesondere monoklonale oder polyklonale Antikoerper, Antiseren, Proteine - insbesondere Protein A, Peptide (sowohl natuerlich vorkommende als auch synthetisch oder gentechnisch hergestellte);

b) Nucleinsaeuren oder deren Derivate oder Fragmente davon, insbesondere einstraengige DNS oder Fragmente davon, doppelstraengige oder partiell doppelstraengige DNS oder Frag mente davon, Oligodesoxyribonucleotide, RNS oder Fragmente davon, oder Oligoribonucleotide oder gentechnisch erzeugte Hybridisationssonden;

c) Proteine mit spezifischer Affinitaet zu einem anderen Biomolekuel, z. B. Streptavidin, Avidin, Lektine oder bestimmte, kopplungsfaehige Enzyme;

d) chemische Verbindungen mit Affinitaet zu einem Biomolekuel, insbesondere Biotin, m-Aminophenylborsaeure oder Cibacron Blue F3GA.

Die Aminoglucosid-Phosphotransferasen werden auch als Aminocyclitol-Phosphotransferasen bezeichnet. Zu ihnen gehoeren die bekannteren Neomycin-Phosphotransferasen I und II (auch Kanamycin-Phosphotransferase I und II, Lividomycin-Phosphotransferase; siehe zur Nomenklatur M. J. Haas, J. E. Dowding, Methods in Enzymology (1975) 43, S. 611 - 617 und Enzyme Nomenclature 1984, Nomenclature Committee, International Union of Biochemistry, Academic Press, New York 1984), die Streptomycin-Phosphotransferase (Abkuerzungen: NPT I bzw. NPT II; APH mit der Bezeichnung der O-Phosphorylierung, z. B. APH 6; siehe dazu K. L. Rinehardt, loc. cit. Von den genannten Aminoglucosid-Phosphotransferasen werden fuer die erfindungsgemaesen enzymmarkierten molekularen Sonden die Neomycin-Phosphotransferase I und II bevorzugt.

Das Enzym kann aus genetisch manipulierten Wirtszellen oder aus Wildstaemmen isoliert werden; diese Verfahren sind z. B. bei B. Reiss, R.

Sprengel, H. Will, H. Schaller, Gene (1984) 30, 211 - 18 und P. Trieu-Cuot und P. Courvalin, J. Antimior. Chemother. (1986) 18, Suppl. C, 93 - 102, beschrieben.

Die Kopplung des Enzyms an die molekulare Sonde kann mittels einer Vielzahl von Methoden und Reagenzien erfolgen. So kann in einer ersten Ausfuehrungsform eine chemische Umsetzung unter Bildung kovalenter Bindungen durchgefuehrt werden, z. B. durch Umsetzung mit bifunktionellen Reagenzien oder durch Kondensation in Gegenwart von Kondensationsmitteln. In einer zweiten Ausfuehrungsform wird die Verbindung von molekularer Sonde und markierendem Enzym durch ionische Bindungen hergestellt, z. B. durch Reaktion in Gegenwart von Polyelektrolyten, Ampholyten oder Reagenzien, die mit beiden Molekuelen ionische Bindungen ausbilden. In einer weiteren Ausfuehrungsform werden molekulare Sonde und markierendes Enzym durch die Bildung von elektrostatischen Wechselwirkungen miteinander verbunden, z. B. durch Modifizierung eines von beiden Molekuelen oder durch ihre Umsetzung mit Tensiden. In einer vierten Ausfuehrungsform werden natuerlich vorkommende, synthetisch hergestellte oder gentechnisch erzeugte Fusionsproteine aus einem Enzym und einem Protein als molekularer Sonde als markierte molekulare Sonde eingesetzt bzw. hergestellt und eingesetzt.

Von der Vielzahl der gegenwaertig zur Verfuegung stehenden Moeglichkeiten zur Kopplung von Enzym und molekularer Sonde werden bevorzugte Verfahren und so hergestellte Produkte beschrieben, wobei jedoch fuer den Fachmann offensichtlich ist, dass ausser den hier genannten andere bifunktionelle Molekuele oder andere Verfahren, insbesondere gentechnische Verfahren oder Konstruktionen, ebenfalls verwendet werden koennen und dabei erfindungsgemaesse, markierte molekulare Sonden erhalten werden. Eine erste, bevorzugte Ausfuehrungsform des erfindungsgemaessen Verfahrens zur Herstellung von markierten molekularen Sonden durch kovalente Verknuepfung von einer Aminoglucosid-Phosphotransferase und einer molekularen Sonde ueber einen Spacer mittels einer chemischen Verbindung besteht darin, als Kopplungsstelle einen Spacer mit einer Laenge zwischen 1 und 150 Atomen zu verwenden, wobei in dieser Kette eine oder mehrere Ringstrukturen enthalten sein koennen.

Durch die Kopplung wird weder die Erkennungsfaehigkeit der Sonde fuer das Biomolekuel noch die Arbeitsfaehigkeit des Enzyms wesentlich beeintraechtigt.

Die Herstellung einer kovalenten Bindung zur Erzeugung einer erfindungsgemaessen, markierten molekularen Sonde kann z. B. durch Gutaraldehyd, p-Benzochinon, Bis-succinimidylester, Bisimidate und/oder Azide erfolgen. Zu den Bis-succinimidylestern zaehlen z. B. (Bis-(2-(succinimidooxycarbonyloxy)ethyl-sulfon, Bis-(sulfosuccinimidyl)-suberat, Disuccinimidyl-suberat, Disuccinimidyl-tartrat, Dithiobis-(succinimidylpropionat), 3,3'-Dithiobis-(sulfosuccinimidyl)-propionat, Ethylenglykolbis-(succinimidylsuccinat), Ethylenglykol-bis-(sulfosuccinimidylsuccinat), Bis-2-sulfosuccinimidoxycarboxyloxy)ethyl-sulfon und Disulfosuccinimidyltartrat. Zu den Bisimidaten gehoeren z. B. Dimethyladipimidat, Dimethyl-3,3'-dithiobis-(propionimidat), Dimethyl-pimelinimidat, Dimethylsuberimidat, usw. Biespiele fuer Azide sind N-5-Azido-2-nitrobenzoyloxysuccinimid, p-Azidophenacylbromid, p-Azidophenylglyoxal, N-(4-Azidophenylthio)-phthalimid, 4,4'-Dithiobis-(phenylazid), 4-Azidophenyl)-1,4-dithio-buttersaeureimid-ethylester, 4-Fluor-3-nitrophenylazid, N-Hydroxysuccinimidyl-4-azidobenzoat, N-Hydroxysuccinimidyl-4-azidosalicylsaeure, 4-Azidobenzoesaeureimid-methylester, 2-Diazo-3,3,3-trifluorpropionylchlorid, p-Nitrophenyl-2-diazo-3,3,3-trifluorpropionat, N-Succinimidyl-6-(4'-azido-2'-nitrophenylamino)hexanoat. Zu dieser Klasse gehoeren auch spaltbare Kopplungsreagenzien, durch deren Einsatz zur Herstellung der erfindungsgemaessen Sonden Produkte erzeugt werden, die in einem Reaktionsschritt des Nachweisverfahrens wieder gespalten werden koennen und damit die Enzymreaktion mit frei beweglichen Enzymen durchgefuehrt werden kann, was einen kinetischen Vorteil beinhaltet.

Zu diesen spaltbaren Kopplungsreagenzien gehoeren z. B. N-Succinimidyl-(4-azidophenyl)-1,3'-dithiopropionat, Sulfosuccinimidyl-2-(m-azido-o-nitrobenzamido)-ethyl-1,3'-dithiopropionat, Sulfosuccinimidyl-(4-azidophenyl-dithio)propionat, Sulfosuccinimidyl-2-(p-azidosalicylamido)ethyl-1,3'-dithiopropionat.

Es ist auch eine direkte kovalente Kopplung von Enzym und molekularer Sonde moeglich. Beispiele hierfuer sind die Kondensation beider Molekuele in Gegenwart von Kondensationsmitteln, z. B. Carbodiimiden.

Fusionsproteine stellen einen Spezialfall fuer die kovalente Verbindung der molekularen Sonden mit den Enzymen dar. Sie werden meistens gentechnisch erzeugt, aber auch synthetisch hergestellt, wobei Sondenprotein und Enzym peptidisch miteinander verknuepft werden.

Bei den gentechnisch hergestellten Fusionsproteinen ist dabei die genetische Information fuer das Sondenprotein und das Enzym benachbart und im durchgaengigen Leseraster auf einem gentechnischen Vektor lokalisiert, wobei sowohl zwischen die fuer die Sonde und das Enzym kodierende DNS weitere fuer Aminosaeuresequenzen (z. B. fuer Proteasespaltorte) kodierende DNS-Fragmente eingefuehrt werden koennen, als auch bestimmte DNS-Fragmente wiederholt auftreten koennen (z. B. Herstellung von Fusionsproteinen aus mehreren Sonden-Molekuelen und/oder Enzym-Molekuelen).

Die auf den Vektoren befindliche Information wird vom Wirtsorganismus, z. B. Bakterien wie E.coli, in eine durchgaengige Aminosaeurensequenz uebersetzt, und das Fusionsprotein kann mikrobiologisch gewonnen werden.

Eine zweite Ausfuehrungsform des erfindungsgemaessen Verfahrens und von erfindungsgemaessen markierten molekularen Sonden ist eine Verbindung von Sonde und markierendem Enzym ueber ionische Gruppen. Dabei koennen Verbindungen verwendet werden, an die sowohl das Enzym als auch die molekulare Sonde ionisch gebunden werden, z. B. Polyethylenimin, Polyelektrolyte oder Ampholyte.

Nach einer dritten Ausfuehrungsform des erfindungsgemaessen Verfahrens und von erfindungsgemaessen markierten molekularen Sonden werden diese durch elektrostatische Wechselwirkungen gekoppelt. Zu den elektrostatischen Wechselwirkungen zaehlen die hydrophob-hydrophob-, Dipol-Dipol-Wechselwirkungen, Bindungen ueber van-der-Waals'sche Kraefte und Wasserstoffbrueckenbindungen.

Die Erfindung bezieht sich auch auf Verfahren zur Herstellung von mittels Aminoglucosid-Phosphotransferasen markierten molekularen Sonden. Das Verfahren besteht darin, an eine molekulare Sonde oder an ein Gemisch molekularer Sonden eine Aminoglucosid-Phosphotransferase zu koppeln. Als Sonden koennen dazu die bereits weiter oben genannten immunreaktiven Sonden, Nucleinsqeuren, Proteine oder chemischen Verbindungen verwendet werden. Als Aminoglucosid-Phosphotransferasen werden Neomycin-Phosphotransferase I und II bevorzugt. Zur Nomenklatur und zu weiteren Varianten zur Durchfuehrung des Verfahrens gilt das gleiche wie oben.

Eine erste Ausfuehrungsform des erfindungsgemaessen Verfahrens zur Herstellung von markierten molekularen Sonden besteht darin, die Aminoglucosid-Phosphotransferase an die molekulare Sonde durch die Herstellung einer direkten kovalenten Bindung oder ueber Kopplungsreagenzien kovalent zu binden. Als Kopplungsreagenzien kommen die bereits genannten in Betracht. Die Reaktionsbedingungen sind von der Art der molekularen Sonde, von der Aminoglucosid-Phosphotransferase und vom Kopplungsreagens abhaengig. Es koennen jedoch auch bereits beschriebene Standard-Protokolle zur Herstellung der Kopplung verwendet werden. Bei den Kopplungsvarianten ist zu beachten, dass fuer bevorzugte Ausfuehrungsformen eine Spacergruppe mit 1 bis 150 Atomen in der Kette und ggf. Ringstrukturen in der Kette verwendet wird, die der markierten molekularen Sonde Gelegenheit gibt, eine moeglichst grosse Anzahl von Substratmolekuelen zu erreichen. Bevorzugt werden Kopplungen mit Glutaraldehyd, p-Benzochinon, Bis-succinimidylestern, Bisimidaten oder Aziden, wie sie bereits beschrieben worden sind.

Eine zweite Ausfuehrungsform des erfindungsgemaessen Verfahrens zur Herstellung enzymmarkierter molekularer Sonden besteht darin, die Kopplung ueber die Ausbildung ionischer Bindungen vorzunehmen. Die Kopplung kann dabei z. B. direkt zwischen einem Protein (Enzym) und einer DNS als molekularer Sonde erfolgen. Bevorzugt werden Varianten, bei denen die Kopplung durch eine oder mehrere chemische Verbindungen erfolgt, zu der/denen beide Reaktionspartner ionische Bindungen ausbilden koennen. Solche Verbindungen sind z. B. Polyethylenimine unterschiedlicher Molmassen, Polyelektrolyte oder Ampholyte, aber auch oligomere oder polymere Verbindungen mit Ladungen im Molekuel.

Eine dritte Ausfuehrungsform des erfindungsgemaessen Verfahrens zur Herstellung enzymmarkierter molekularer Sonden besteht darin, zur Kopplung elektrostatische Wechselwirkungen von Enzym und molekularer Sonde zu nutzen. Solche Wechselwirkungen koennen hydrophob-hydrophob oder Dipol-Dipol sein oder ueber van-der-Waals'sche Kraefte oder Wasserstoffbrueckenbindungen erfolgen.

Eine vierte Ausfuehrungsform des erfindungsgemaessen Verfahrens zur Herstellung enzymmarkierter molekularer Sonden besteht in der gentechnischen oder synthetischen Herstellung von Fusionsproteinen, bei denen Sonde und Enzym direkt miteinander verbunden sind. Allgemeine Vorschriften zur Herstellung von Vektoren und Fusionsproteinen finden sich bei T. Maniatis, E. F. Fritsch, J. Sambrook, Molecular Cloning, Cold Spring Harbor Laboratory, 1982, S. 97 ff. und 247 ff. Ein genaues Protokoll zur Verwendung und Herstellung eines neuartigen Fusionsproteins aus Neomycin-Phosphotransferase II und Protein A, das ebenfalls ein neuartiges Verfahren darstellt, wird in den folgenden Beispielen beschrieben.

Das erfindungsgemaesse Verfahren zum Nachweis von Biomolekuelen mittels enzymmarkierter molekularer Sonden stellt ein neues Prinzip des Nachweises von Biomolekuelen dar. Die Verwendung von molekularen Sonden mit daran gekoppelten Enzymen und der Nachweis des umgesetzten Substrates an einer festen Phase hat eine grosse Vielfalt an Ausfuehrungsformen. Das Verfahren kann auf eine Vielzahl von Biomolekuelen angewendet werden und ist deshalb universell anwendbar. Bei der Anwendung wird ein Verstaerkungsfaktor von mindestens 10 erreicht, er liegt im allgemeinen jedoch wesentlich darueber. Die erreichbare Verstaerkung ist von den eingesetzten Stoffen und von der gewaehlten Verfahrensvariante abhaengig und kann nur fuer den speziellen Fall exakt angegeben werden. Durch die unterschiedlichen Ausfuehrungsformen besteht beim Arbeiten nach bestimmten Varianten die Moeglichkeit, die verwendeten Biomolekuele mit mehreren Sonden nachzuweisen, d. h. die Blots sind z. B. wiederverwendbar. Die Ausführung des erfindungsgemaessen Verfahrens ist aufgrund seiner allgemeinen Konzeption nicht auf die beschriebenen Verfahrensvarianten beschränkt.

Durch die bevorzugte Ausfuehrungsform des erfindungsgemaessen Verfahrens zum Nachweis von Biomolekuelen mittels an Aminoglucosid-Phosphotransferasen gekoppelter molekularer Sonden ist es moeglich, Biomolekuele durch molekulare Sonden mit hoeherer Empfindlichkeit als bisher bekannt nachzuweisen. Darueber hinaus kann der Nachweis der Biomolekuele an einer zweiten, getrennten festen Phase durchgefuehrt werden, wodurch das Biomolekuel nicht geschaedigt wird und damit wiederverwendbar bleibt. Ein solches indirektes Nachweisverfahren bei Nachweisen in der Genetik, beim Nachweis genetisch bedingter Krankheiten beim Menschen und von Virusspaltprodukten hat grosse Bedeutung, da der Nachweis mit mehreren Sonden gefuehrt werden kann, die biologischen Molekuele durch schonende Inkubationen nicht geschaedigt werden und schliesslich ein hoher Verstaerkungsfaktor erreicht wird, der bisher nicht erzielt werden konnte.

Die fuer die bevorzugte Ausfuehrungsform des erfindungsgemaessen Verfahrens hergestellten markierten molekularen Sonden stellen neue Produkte mit ungewoehnlichen Eigenschaften dar und koennen besonders in Nachweisverfahren fuer Biomolekuele verwendet werden.

Die bevorzugt verwendeten Enzyme, die Aminoglucosid-Phosphotransferasen, zeichnen sich durch hohe Bestaendigkeit in allen Nachweisschritten aus, d. h. die erfindungsgemaessen markierten molekularen Sonden koennen in Hybridisationsverfahren bei Temperaturen von 65 °C eingesetzt werden. Durch die Verwendung dieser Sonden ist beim autoradiographischen Nachweis ein sehr hoher Verstaerkungsfaktor erreichbar. Bisher ist eine solche Verstaerkung mit an Enzyme gekoppelten Sonden nicht erreicht worden.

So wurden z. B. beim Protein-Nachweis 10 pg sicher nachgewiesen.

Beim bisherigen Nachweis von Proteinen z. B. betrugen mit $^{125}$I-Protein A die Autoradiographiezeiten fuer Mengen im Bereich von 10 ng 1 - 3 Tage bei -70 °C. Mit dem erfindungsgemaessen Verfahren sind im o. g. Falle Autoradiographiezeiten von 30 min bis 1 Stunde bei Zimmertemperatur fuer starke Signale ueblich.

Anschliessend wird die Erfindung an Beispielen naeher erlaeutert:

Verwendete Pufferloesungen:

PBS:

8 g NaCl, 0,2 g $KH_2PO_4$, 2,9 g $Na_2HPO_4$. 12 $H_2O$, 0,2 g KCl werden in Wasser geloest und auf 1 l aufgefuellt, pH = 7,4

TBS:

0,15 M NaCl, 50 mM Tris(hydroxymethyl)-aminomethan (Tris), pH = 7,4

GET:

2,5 mM Tris/HCl, pH = 8,0, 5 mM Glucose, 1 mM Ethylendiamintetraessigsaeure, Tetranatriumsalz (EDTA)

Lysepuffer:

125 mM Tris/HCl, pH = 6,8, 2 % Natriumdodecylsulfat (SDS), 20 % Glycerin, 10 % $\beta$-Mercaptoethanol

Elutionspuffer:

20 mM Tris/HCl, pH = 7,4, 10 mM Dithiothreitol, 0,1 % SDS

Loesepuffer:

in 100 ml 20 mM Tris/HCl, pH = 8,8, werden 48g Harnstoff geloest und dazu 1 % Nonidet NP 40 und 10 % -Mercaptoethanol gegeben.

Enzympuffer:

20 mM Tris/HCl pH 7,2, 125 mM NH$_4$Cl, 20 mM MgCl$_2$ 10 mM Dithiothreitol

Beispiel 1

Kopplung von polyklonalem Antikoerper an Kanamycin-Phosphotransferasen

1 ml Anti-Humanserumalbumin-Antiserum (Kaninchen) mit 0,9 % NaCl wird mit 10 mg Benzochinon in 0,3 ml Ethanol 1 Stunde bei Zimmertemperatur zur Reaktion gebracht. Danach wird ueberschuessiges Benzochinon mittels einer Sephadex G25-Saeule abgetrennt und der Komplex Benzochinon-Antikoerper fraktioniert. Die einzelnen Fraktionen werden in einem Immuntest gegen Humanserumalbumin (HSA) geprueft. Die gesuchte Fraktion wird mit 1 mg Kanamycin-Phosphotransferase in 1 ml 1 M Bicarbonat-Puffer (pH-Wert 9) ueber Nacht bei Zimmertemperatur im Dunkeln zur Reaktion gebracht.

Das Reaktionsprodukt wird ueber Nacht gegen 0,9 % NaCl-Loesung dialysiert und so verwendet. Es kann nach der Dialyse auch in Aliquots aufgeteilt und bei -20° C gelagert werden, oder das Produkt Antikoerper-Benzochinon-Kanamycin-Phosphotransferase kann sofort zur immunologischen Reaktion eingesetzt werden.

Beispiel 2

Immunologischer Nachweis von HSA durch einen Kanamycin-Phosphotransferase gekoppelten Antikoerper mit Nachweis des umgesetzten Substrates auf einer festen Phase III

Ein Gemisch aus verschiedenen Markerproteinen und HSA wurde in einem Polyacrylamid-Gel (PAA-Gel) elektrophoretisch aufgetrennt und auf Nitrocellulosefilter durch Elektrotransfer uebertragen (Blot). Dieser Blot wird mit einem Kanamycin-Phosphotransferase-gekoppelten Antikoerper nach Beispiel 1 in TBS (durch Tris-(hydroxymethyl)-aminomethan gepufferte Kochsalzloesung von 0,9 %) und 1 % Gelatine 4 Stunden bei Zimmertemperatur inkubiert (Verduennung 1 : 500). Danach wird der Blot mit dem angelagerten, am Enzym gekoppelten Antikoerper 4 mal 15 Minuten in TBS gewaschen und mit einem 1 % Agarosegel (im Reaktionspuffer hergestellt) mit 20 mMol Tris/HCl, pH = 7,2, 125 mMol NH$_4$Cl, 1 mMol Dithioerythrol, 20 mMol MgCl$_2$, 2 mMol Adenosintriphosphat (ATP), das 1 μCi gamma-$^{32}$P-ATP/ml und 20 μg/ml Kanamycin enthaelt, 30 Minuten bei 37° C inkubiert. Das Agarosegel wird anschliessend auf Phosphorcellulose-Flaechentraeger aufgelegt und das Kanamycin auf die Phosphorcellulose uebertragen (Zellstoffstapel 0,5 kg Gewicht/3 Stunden/Zimmertemperatur). Der Nachweis des mit $^{32}$P markierten Kanamycins erfolgt durch Autoradiographie des Phosphorcellulose-Flaechentraegers (2 Stunden, Zimmertemperatur).

Beispiel 3

Immunologischer Nachweis von HSA mittels eines Kanamycin-Phosphotransferase-markierten Antikoerpers auf eine feste Phase II

Humanserum wird in einem 1,5 % Agarosegel elektrophoretisch aufgetrennt. Das Gel wird mit einem an Kanamycin-Phosphotransferase gekoppelten Antikoerper 1 Stunde bei Zimmertemperatur mit TBS mit 2 % Rinderserumalbumin (RSA) (Verduennung 1 : 500) inkubiert und danach 1 Stunde mit TBE gewaschen.

Das Gel wird zunaechst in einem Reaktionspuffer wie in Beispiel 2, bestehend aus 20 mMol Tris/HCl, pH-Wert = 7,2, 125 mMol NH$_4$Cl, 1 mMol Dithioerythrol, 20 mMol MgCl$_2$ und 2 mMol ATP, das 1 μCi $^{32}$P-gamma-ATP enthaelt, eine halbe Stunde inkubiert und danach mit einem Traeger nach DD-WP-Anmeldung C 07 G/296.754-2, der aus Cellulose, die mit Cyanurchlorid und anschliessend mit Sulfanilsaeure umgesetzt wurde, besteht und wo an dieses Produkt ionisch Kanamycin gebunden wurde, in Kontakt gebracht und 30 Minuten bei 37° C umgesetzt. Nach Waschung (30 Minuten in Wasser) des Traegers II mit dem Substrat und dem phosphorylierten Substrat wird dieser 1 Stunde bei Zimmertemperatur autoradiographiert und so das $^{32}$P-markierte Kanamycin auf den Traeger II nachgewiesen, wodurch das Vorhandensein von HSA im Trenngel bewiesen wird.

Beispiel 4

Kopplung von Protein A an Neomycin-Phosphotransferase II mittels Glutaraldehyd

a) Elektrophoretische Reinigung von NPT II aus E.coli Lysaten

Das Zellpellet von 10 ml E. coli (NPT II ueberexprimierend) Zellkultur (pRK 89) wird in 1 ml Lysepuffer aufgenommen, 1 Minute beschallt, 3 Minuten gekocht und erneut beschallt. Zellreste werden 5 Minuten bei 4° C abzentrifugiert. Das Zellysat wird auf ein praeparatives Polyacrylamid/SDS-Gel aufgetragen und elektrophoretisch aufgetrennt.

Das Gel wird mit eiskaltem Wasser abgespuelt und in eine kalte Loesung von 0,25 M KCl gelegt, bis die Proteinbanden gegen einen dunklen Untergrund gut zu erkennen sind. Die NPT II-Bande wird mit einem Skalpell herausgeschnitten und das Gel in kleine Wuerfel zerteilt. Zur Elution des Proteins aus dem Gel wird dieses mit Elutionspuffer bedeckt und zunaechst 3 Stunden bei Zimmertemperatur geschuettelt. Nach Wechsel des Puffers wird ueber Nacht nochmals eluiert.

Die so erhaltenen Loesungen werden bei 6 000 rpm zentrifugiert und der Ueberstand mit dem vierfachen Volumen kaltem Aceton versetzt und 2 bis 3 Stunden bei -20° C stehengelassen. Danach wird bei 0° C und 4 000 rpm zentrifugiert.

Der Niederschlag wird mit einer Loesung aus 1 Teil 20 mMol Tris/HCl, pH = 7,4, und 4 Teilen Aceton kalt gewaschen und erneut zentrifugiert. Das Pellet wird in 200 µl Loesepuffer geloest und anschliessend gegen 20 mM Natriumphosphatpuffer, pH = 7, ueber Nacht dialysiert (500 ml, 2 Wechsel, 4° C). Das Endvolumen betraegt ca. 200 µl und enthaelt ungefaehr 200 µg NPT II mit geringen Restverunreinigungen an E. coli Proteinen.

b) Kopplung mit Glutaraldehyd

400 µg Protein A (Pharmacia AB) und 400 µg nach a) gereinigte NPT II werden gemeinsam in einem Dialyseschlauch von 1,5 ml Volumen gegen PBS dialysiert (16 Stunden, 4° C, 800 ml, 2 Wechsel). Nach Umfuellen der dialysierten Loesung in ein Glasroehrchen werden 6 µl Glutaraldehyd (25 %ige Loesung, Serva) zugegeben und 2 Stunden bei Zimmertemperatur unter leichtem Schuetteln umgesetzt. Anschliessend wird dieses Produkt wieder in den Dialyseschlauch gefuellt und zunaechst gegen PBS (4 Stunden bei 4° C) und danach 16 Stunden gegen TBS (4° C, 800 ml, 2 Wechsel) dialysiert. Nach der Dialyse wird das Reaktionsprodukt in ein Glasroehrchen umgefuellt und bei 4° C gelagert. Auf einer Kontroll-Elektrophorese (10 % SDS/Polyacrylamid-Gel) werden vor und nach der Kopplung entnommene Proben von 20 µl aufgetrennt und analysiert (nach dem Festphasen-NPT-II-Test nach DD-WP 254.029). Es zeigt sich, dass saemtliches NPT II und Protein A zu hochmolekularen Produkten vernetzt wurde. Die Enzymaktivitaet bleibt nach der Kopplung nahezu vollstaendig erhalten. Im Falle einer unvollstaendigen Kopplung kann nochmals mit Glutaraldehyd vernetzt werden bzw. nicht umgesetztes Protein A mittels einer kleinen Saeule an Sephadex G-50 abgetrennt werden.

Das so hergestellte Produkt wird zum Nachweis von mit Antikoerpern markierten Biomolekuelen eingesetzt.

Beispiel 5

Kopplung von Neomycin-Phosphotransferase II an Anti-Kartoffelvirus-Immunoglobuline mittels Glutaraldehyd

400 µg nach Beispiel 4a hergestellte und gereinigte NPT II werden zusammen mit 1 mg Anti-Kartoffelvirus-Immunoglobulinen in einem Dialyseschlauch gegen PBS (16 Stunden, 4° C, 800 ml, 2 Wechsel) bei einem Schlauchvolumen von 1 ml dialysiert. Nach Umfuellen der dialysierten Probe in ein Glasroehrchen werden 5 µl Glutaraldehyd (25 %ige Loesung, Serva) zugegeben und 2 Stunden unter leichtem Schuetteln bei Zimmertemperatur umgesetzt. Anschliessend wird der Kopplungsansatz in den Dialyseschlauch zurueckgefuellt und zunaechst gegen PBS (4° C, ueber Nacht) und danach gegen TBS (4° C, 800 ml, 2 Wechsel, 16 Stunden) dialysiert. Anschliessend wird das Reaktionsprodukt in ein Glasroehrchen umgefuellt und bei 4° C gelagert. Nach Reifung (1 Tag) ist das Kopplungsprodukt, ein mittels NPT II markierter Antikoerper, der ueber Glutaraldehyd gekoppelt wurde, zum immunologischen Nachweis von Kartoffelviren aus Pflanzensaft im Picogrammbereich einsetzbar.

Beispiel 6

Konstruktion eines Protein A-NPT II-Fusionsproteinvektors, Expression und Gewinnung des Fusionsproteins

In den Multi-cloning-site des Protein A-Fusionsprotein-Vektors pRIT 2 (B. Nilson, L. Abrahmsen, N. Uhlen, EMBO Journal 4 (4), (1985) S. 1 075 bis 1 080) wird das NPT II-Gen (Tn5) (S. Brenner, Nature 329 (1987), S. 21) aus E.coli nach klassischer Klonierungsmethode (BamHI, EcoRI-Spaltung, T4-Ligasereaktion) im offenen Leseraster eingesetzt. Die Transformation von E.coli (NF1) und die Selektionierung des entsprechenden Klones ueber Ampicillinresistenz erfolgten nach den ueblichen Methoden (T. Maniatis, E. F. Fritsch, J. Sambrock, Molecular Cloning, Cold Spring Harbor-Laboratory 1982).

Die Anzucht des Protein A-NPT II-Fusionsprotein Klones erfolgt im Fluessigmedium NB 1 (Immunpraeparate Berlin, DDR) nach ueber Nacht durchgefuehrter Vorkultur ungefaehr 6 Stunden bei 30°C. Nach Temperaturerhoehung auf 42°C wird das Fusionsprotein exprimiert ($P_R$ promoter des Phagen $\eta$). Nach 2 Stunden werden die Zellen abzentrifugiert, 1 mal mit GET-Puffer gewaschen, in GET resuspendiert und in Portionen zu je $10^{10}$ Zellen in Eppendorf-Roehrchen abzentrifugiert. Die Zellen werden portioniert bei -20°C gelagert und stehen zur Gewinnung des Fusionsproteins zur Verfuegung. Zur Gewinnung des Fusionsproteins werden $10^{10}$ Zellen im Eppendorf-Roehrchen mit 1 ml 20 mM Tris/HCl-Puffer pH 6,8, bei 0°C resuspendiert und 5 mal kurz von aussen beschallt. Nach Abkuehlung in Eis wird erneut 3 mal kurz beschallt. Die Zellreste werden bei 4°C 5 Minuten abzentrifugiert. Der Ueberstand wird vorsichtig abpipettiert und in ein zweites Eppendorf-Roehrchen ueberfuehrt. Das Zellysat wird in dieser Form fuer Nachweisreaktionen verwendet oder bei -20°C gelagert.

## Beispiel 7

### Vernetzung eines gentechnisch erzeugten Protein A-NPT II-Fusionsproteins mittels Glutaraldehyd

Es wird ein Zellysat wie in Beispiel 6 hergestellt. Von diesem Lysat werden 50 μl fuer die Kontroll-Elektrophorese in ein Eppendorf-Roehrchen abpipettiert. Das Lysat wird in einen Dialyseschlauch gefuellt und gegen PBS (4°C, 16 Stunden, 800 ml, 2 Wechsel) dialysiert. Nach Umfuellen des dialysierten Lysates in ein Glasroehrchen wird dieses mit 3 μl Glutaraldehyd (25 %ige Loesung, Serva) versetzt und 2 Stunden bei Zimmertemperatur unter leichtem Schuetteln inkubiert. Danach wird der Reaktionsansatz zurueck in den Dialyseschlauch gefuellt und zunaechst 3 Stunden gegen PBS (4°C) und danach ueber Nacht gegen TBS (4°C, 800 ml, 2 Wechsel) dialysiert. Der dialysierte Kopplungsansatz wird aus dem Dialyseschlauch entnommen und in einem Glasroehrchen bei 4°C gelagert. 50 μl werden fuer die Kontroll-Elektrophorese entnommen.

Auf einer Kontroll-Elektrophorese (10 % SDS/Polyacrylamid (PAA)-Gel) werden die entnommenen Proben aufgetrennt und durch den Festphasen-NPT II-Test mittels $^{32}P$-$\gamma$-ATP und Kanamycin analysiert. Es zeigt sich, dass waehrend der Kopplung saemtliches Protein A-NPT II-Fusionsprotein (Molmasse ungefaehr 60 kD) zu hochmolekularen Kopplungsprodukten vernetzt wurde. Die Enzymaktivitaet des Kopplungsproduktes ist voll erhalten. Das erhaltene Produkt kann sofort zum Nachweis von Antikoerper-markierten Antigenen eingesetzt werden.

## Beispiel 8

### Kopplung eines gentechnisch erzeugten Protein A-NPT II-Fusionsproteins mit weiteren NPT II-Molekuelen mittels Glutaraldehyd

Es wird ein Zellysat wie in Beispiel 6 hergestellt und diesem 100 μg nach Beispiel 4 hergestellte und elektrophoretisch gereinigte NPT II zugesetzt. Dieses Gemisch wird 16 Stunden gegen PBS dialysiert (Dialyseschlauch 1,5 ml Volumen, 800 ml, 2 Wechsel, 4°C). Das dialysierte Gemisch wird in einem Glasroehrchen mit 3 μl Glutaraldehyd (25 %ige Loesung, Serva) versetzt und unter Schuetteln 2 Stunden bei Zimmertemperatur inkubiert. Die Vollstaendigkeit der Kopplung wird durch Elektrophorese und Festphasen-NPT II-Test kontrolliert.

Nach der Kopplung wird erneut zunaechst gegen PBS und danach gegen TBS dialysiert (jeweils bei 4°C, 800 ml, 2 Wechsel). Das dialysierte Kopplungsprodukt wird bei 4°C gelagert; es wird zum Nachweis von Antikoerper-markierten Antigenen eingesetzt.

## Beispiel 9

### Kopplung von Streptavidin und NPT II mittels Glutaraldehyd

100 μg Streptavidin und 400 μg nach Beispiel 4 hergestellte NPT II werden wie in Beispiel 8 gegen PBS (1 ml Schlauchvolumen) dialysiert und danach mit 5 μl Glutaraldehyd (25 %ige Loesung, Serva) versetzt und eine Stunde bei Zimmertemperatur unter leichtem Schuetteln umgesetzt. Der Reaktionsansatz wird erneut zunaechst gegen PBS und danach gegen TBS dialysiert (jeweils 4°C, 4 Stunden, 800 ml, 2 Wechsel). Das dialysierte Produkt wird bei 4°C gelagert. Die Vollstaendigkeit der Kopplung wird mittels Kontroll-Elektrophorese und Festphasen-NPT II-Test ueberprueft.

Das Kopplungsprodukt wird zum Nachweis von Biotin-markierten Biomolekuelen (z. B. biotinylierter DNS) eingesetzt.

Beispiel 10

Kopplung von NPT II und Protein A mittels eines Toluylendiisocyanat-Praepolymeren

a) Herstellung des Kopplungsreagenzes

17,4 g frisch destilliertes Toluylendiisocyanat-2,4 werden unter einer Stickstoffatmosphaere auf 50° C erwaermt. Unter Ruehren werden bei 50 bis 55° C 30 g Polyethylenglykol 600 ueber Ionenaustauscher gereinigt und 3 Stunden bei 80° C/0,1 Torr entwaessert) und 0,05 g Cyanurchlorid langsam zugetropft (etwa 30 Minuten). Nach Beendigung der Zugabe wird noch 4 Stunden bei 60° C geruehrt. Danach wird bei 50° C/0,1 Torr entgast (Isocyanat-Aequivalent: 453).

100 mg des Praepolymeren werden in 500 mg Aceton geloest. Nach vollstaendiger Loesung werden 0,3 ml dieser Loesung unter schnellem Ruehren in 700 ml Wasser gespritzt. Die so erhaltene Emulsion wird sofort verwendet.

b) Kopplung

800 µl nach Beispiel 4 erhaltenen Lysates mit NPT II (100 µg) werden mit 150 µg Protein A in 150 µl Wasser in einem Eppendorf-Roehrchen vermischt. Zu dieser Loesung werden 200 µl des oben hergestellten Kopplungsreagenzes zugegeben und 1 Stunde leicht geschuettelt. Alle 15 Minuten wird mit einem Virtex gut durchmischt. Nach 1 Stunde Reaktionszeit werden erneut 100 µl frisch hergestelltes Kopplungsreagenz zugesetzt und eine weitere Stunde umgesetzt. Danach werden zum Abstoppen der Reaktion 150 µl Lysin-Loesung (200 µg) zugesetzt und 30 Minuten bei Zimmertemperatur geschuettelt. Die Vollstaendigkeit der Kopplung wurde durch Kontroll-Elektrophorese und Festphasen-NPT II-Test nachgewiesen. Das hochmolekulare Kopplungsprodukt weist eine vollstaendige Enzymaktivitaet auf.

Es wird zum Nachweis von Antikoerper-markierten Biomolekuelen eingesetzt.

Beispiel II

Biologische Vernetzung eines Protein A-NPT II-Fusionsproteins

50 µl eines nach Beispiel 6 hergestellten Zellysates mit Protein A-NPT II-Fusionsprotein werden mit 5 µl Kaninchen-Anti-Maus-Antiserum vermischt und bei 4° C ueber Nacht inkubiert. Der sich bildende Komplex aus Protein A-NPT II-Fusionsprotein und Kaninchen-Anti-Maus-Antikoerper wird als 2. Antikoerper nach dem Nachweis eines Antigens mit dem 1. Antikoerper (Maus) verwendet. Dadurch wird der enzymatische Nachweis (NPT II) gebracht.

Beispiel 12

Kopplung eines Lektins an Neomycin-Phosphotransferase II mittels Glutaraldehyd

200 µg eines Phaseolus-vulgaris-Lektins werden zusammen mit 400 µg nach Beispiel 4 erhaltenem, gereinigtem NPT II gegen PBS dialysiert (1 ml Schlauchvolumen, 4° C, 800 ml, 2 Wechsel, ueber Nacht). Der Inhalt des Dialyseschlauches wird in ein Glasroehrchen gefuellt und mit 5 µl Glutaraldehyd (25 %ige Loesung, Serva) versetzt und 1 Stunde bei Zimmertemperatur unter leichtem Schuetteln umgesetzt. Der Reaktionsansatz wird dann erneut gegen PBS und anschliessend gegen TBS dialysiert (jeweils 4° C, 4 Stunden, 800 ml, 2 Wechsel). Das dialysierte Reaktionsprodukt wird bei 4° C gelagert. Die Vollstaendigkeit der Kopplung und die enzymatische Aktivitaet werden mittels Kontroll-Elektrophorese und Festphasen-NPT II-Test ueberprueft. Das Kopplungsprodukt dient zum Nachweis entsprechender Kohlehydrate an Glykoproteinen.

Beispiel 13

Immunologischer Nachweis von Humanserumalbumin nach Protein-Blot auf Nitrocellulose unter proteinfreien Blockbedingungen (Verwendung eines Protein A-NPT II-Fusionsproteins)

Eine Verduennungsreihe aus Proteinen mit Humanserumalbumin (HSA) im Nanogramm-Picogramm-Bereich wurde im SDS-PAA-Gel elektrophoretisch aufgetrennt und mittels Elektrotransfer auf Nitrocellulose-Membran transferiert. Anschliessend wurde die Nitrocellulose-Membran (Blot) 30 Minuten an der Luft getrocknet und danach in TBS mit 1 Vol.-% Tween 20 eine Stunde bei 37°C geblockt. Eine weitere Blockierung erfolgt anschliessend in TBS mit 0,2 Vol.-% Tween 20 innerhalb von 30 Minuten (Block I).

Die Inkubation mit dem Antiserum Anti-HSA-Immunoglobulin (Kaninchen) erfolgte in einer Verduennung von 1 : 200 eine Stunde bei Zimmertemperatur. Danach wurde eine Stunde mit TBS mit 0,1 Vol.-% Tween 20 bei Zimmertemperatur gewaschen (6 Wechsel). Nach erneuter Blockung mit TBS und 0,2 Vol.-% Tween 20 (30 Minuten bei Zimmertemperatur) (Block II) wurden 50 ul des wie Beispiel 6 hergestellten Lysates mit dem Fusionsprotein aus Protein A und NPT II in ein Volumen von 10 ml gegeben und eine Stunde bei Zimmertemperatur unter leichtem Schuetteln inkubiert. Anschliessend werden die Nitrocellulose-Membranen (Hybond C, Amersham International) eine Stunde mit TBS gewaschen (6 Wechsel).

Eine nach DD-WP 254.012 aus mit Cyanurchlorid aktivierter Cellulose und Sulfanilsaeure hergestellter und mit Kanamycin beladener Traeger wird auf eine Glasplatte gelegt und mit dem Enzympuffer, der $^{32}P$-$\gamma$-ATP mit einer Konzentration von 2 Mio. cpm/ml (spezifische Aktivitat 150 TBq/mMol) enthaelt, getraenkt. Die oben erhaltenen Blots auf Nitrocellulose werden mit der beim Elektrotransfer gelseitigen Seite auf den feuchten, das Kanamycin tragenden Traeger gelegt. Anschliessend wird die Nitrocellulose-Membran nochmals von oben mit dem $^{32}P$-$\gamma$-ATP enthaltenden Enzympuffer getraenkt und schliesslich mit einer Glasplatte gleicher Groesse abgedeckt. Dieses System wird allseitig mit PE-Folie verschlossen und zunaechst eine Stunde bei 37°C inkubiert, wobei waehrend dieser Zeit die Glasplatten mittels eines Gewichtes leicht zusammengedrueckt werden. Nach der Inkubation wird aus dem System der Kanamycin tragende Traeger entnommen und eine Stunde mit bidest. Wasser gewaschen. Die Nitrocellulose-Membran wird sofort erneut auf einen Kanamycin enthaltenden Traeger aufgelegt, mit $^{32}P$-$\gamma$-ATP enthaltendem Enzympuffer befeuchtet und ueber Nacht bei 37°C inkubiert. Anschliessend wird mit bidest. Wasser gewaschen. Die Auswertung erfolgt durch Autoradiographie der im Kontakt verwendeten, mit Kanamycin beladenen Traeger. Die Nitrocellulose-Membranen werden nach Waschung mit bidest. Wasser (frei von $^{32}P$-$\gamma$-ATP) zur Kontrolle mit autoradiographiert.

Durch die Autoradiogramme der im Kontakt verwendeten Traeger wird die Lage der HSA-Banden bis in den Picogramm-Bereich als geschwaerzte Banden auf dem Roentgenfilm dargestellt (Exposition: 30 Minuten bei Zimmertemperatur, Roentgenfilm HS 11, VEB ORWO Wolfen).

Da proteinfreie Blockbedingungen verwendet wurden, kann das Protein auf der Nitrocellulose-Membran mit Ponceau S angefaerbt werden (Faerbung: 2 Minuten, Entfaerbung mit Wasser: 5 Minuten). Zur Auswertung wird die Autoradiographie des im Kontakt verwendeten Traegers ueber die angefaerbten Nitrocellulose-Blots gelegt, so dass die Banden genau zugeordnet werden koennen.

Beispiel 14

Immunologischer Nachweis von HSA nach Protein-Blotting auf Nitrocellulose unter Protein-Blockungs-Bedingungen

Es wird ebenso wie in Beispiel 13 verfahren, jedoch wird als Block I TBS mit 0,5 Gew.-% Gelatine (1 Stunde) und TBS mit 0,25 Gew.-% Gelatine (30 Minuten) sowie als Block II TBS mit 0,25 Gew.-% Gelatine (30 Minuten) verwendet.

Die Auswertung erfolgt wie in Beispiel 13 durch Autoradiographie der im Kontakt verwendeten Traeger.

Beispiel 15

Immunologischer Nachweis von $\alpha$-Fetoprotein im Tuepfeltest auf Nitrocellulose

Eine Verduennungsreihe von $\alpha$-Fetoprotein (AFP) wird auf einen Streifen Nitrocellulose-Membran (Hybond C, Amersham Int.) aufgetuepfelt und bei Zimmertemperatur 30 Minuten trocknen gelassen. Die Blockierung erfolgt im Block I mit TBS und 1 % Tween 20 1 Stunde bei 37°C und danach in TBS mit 0,2 % Tween 20 30 Minuten bei Zimmertemperatur. Zunaechst wird mit einem monoklonalen Antikoerper (Maus) gegen HSA (Verduennung 1:1000) inkubiert, danach mit TBS + 0,1 % Tween 20 gewaschen, erneut mit TBS + 0,2 % Tween 20 geblockt und mit einem zweiten Antikoerper (Kaninchen-Anti-Maus) 1 Stunde inkubiert (Verduennung 1:500). Nach Waschung mit TBS + 0,1 % Tween 20 eine Stunde bei Zimmertemperatur wird erneut mit TBS + 0,2 % Tween 20 geblockt.

Danach werden 35 $\mu$l E.coli-Lysat, das nach Beispiel 6 hergestellt worden ist, und das das Fusionsprotein aus Protein A und NPT II enthaelt, zugegeben und 1 Stunde bei Zimmertemperatur inkubiert. Nach Waschen mit TBS (eine Stunde bei Zimmertemperatur) wird die Nitrocellulose-Membran mit dem Kanamycin-beladenen Traeger (hergestellt nach DD-WP 254.012) in Kontakt gebracht und der Nachweis wie in Beispiel 13 beschrieben durchgefuehrt. Die Nachweisgranze liegt im Picogramm-Bereich.

## Beispiel 16

Nachweis von Kartoffel-Virus nach Tuepfeln von Pflanzensaeften auf Nitrocellulose mittels NPT II-gekoppelten Antikoerpern (Nachweis auf Traegern nach DD-WP 254.029)

a) Kopplung von Neomycin-Phosphotransferase II an Anti-Kartoffelvirus-Immunoglobuline mittels Glutaraldehyd

440 µg nach Beispiel 4a hergestellte und gereinigte NPT II werden zusammen mit 1 mg Anti-Kartoffelvirus-Immunoglobulinen in einem Dialyseschlauch gegen PBS (16 Stunden, 4 °C, 800 ml, 2 Wechsel) bei einem Schlauchvolumen von 1 ml dialysiert.Nach Umfuellen der dialysierten Probe in ein Glasroehrchen werden 5 µl Glutaraldehyd (25 %ige Loesung, Serva) zugegeben und 2 Stunden unter leichtem Schuetteln bei Zimmertemperatur umgesetzt. Anschliessend wird der Kopplungsansatz in den Dialyseschlauch zurueckgefuellt und zunaechst gegen PBS (4 °C, ueber Nacht) und danach gegen TBS (4 °C, 800 ml, 2 Wechsel, 16 Stunden) dialysiert. Das Reaktionsprodukt wird in Glasroehrchen abgefuellt.

b) Durchfuehrung des Nachweises

Eine Verduennungsreihe Kartoffelblatt-Presssaft wird auf einen Streifen Nitrocellulose-Membran aufgetuepfelt und 30 Minuten eintrocknen lassen. Die so behandelte Membran wird 1 Stunde bei 37 °C in TBS + 1 % Tween 20 und anschliessend 30 Minuten bei Zimmertemperatur in TBS + 0,2 % Tween 20 geblockt. Danach wird das oben hergestellte Kopplungsprodukt in einer Verduennung von 1 : 200 zugesetzt und 1 Stunde inkubiert. Nach der Waschung mit TBS erfolgt der radioaktive Nachweis auf der mit Kanamycin beladenen Traegerschicht nach dem Kontaktverfahren analog Beispiel 13. Die Nachweisgrenze liegt im Picogramm-Bereich.

## Beispiel 17

Nachweis von Kartoffelvirus aus Pflanzensaeften nach Tuepfeln auf Nitrocellulose mittels Protein A-NPT II-Fusionsprotein (Kontakttraeger P 81)

Es wird eine Verduennungsreihe mit Kartoffelblatt-Presssaft wie in Beispiel 16 hergestellt. Nach der Blockierung wird jedoch eine Stunde mit Anti-Kartoffelvirus-Immunoglobulinen (Kaninchen) (Verduennung 1 : 1 000) inkubiert. Nach Waschen mit TBS + 0,1 % Tween 20 wird erneut mit TBS + 0,2 % Tween 20 geblockt und danach 50 µl Lysat nach Beispiel 6 zugegeben und 1 Stunde inkubiert. Nach der Waschung mit TBS wird das Kontaktverfahren analog zu Beispiel 13b zum Nachweis durchgefuehrt.

Als Kanamycin-beladener Traeger wird jedoch Phosphorcellulose (P 81, Whatman, England) eingesetzt. Im Enzympuffer wird eine spezifische Aktivitaet des $^{32}P$-$\gamma$-ATP von 10 Mio cpm/ml verwendet.

Die Auswertung erfolgt durch Autoradiographie der Phosphorcellulose-Traeger nach Waschung mit Wasser (1 Stunde). Die Empfindlichkeit liegt wiederum im Picogramm-Bereich.

## Beispiel 18

Immunologischer Nachweis von HSA nach Tuepfeln auf Nylon-Membranen mittels Protein A-NPT II-Fusionsprotein (Kontakttraeger: Kanamycin-beladene Phosphorcellulose)

Eine Verduennungsreihe HSA wird auf einen Streifen Nylon-Membran (Hybond N, Amersham Int.) aufgetuepfelt und 30 Minuten trocknen gelassen. Die Blockierung I erfolgt 2 Stunden in TBS mit 3 % Rinderserumalbumin (RSA) und danach 30 Minuten in TBS mit 1 % RSA. Die Blockierung II erfolgt 30 Minuten in TBS mit 1 % RSA. Das Gesamtverfahren erfolgt ebenso wie in Beispiel 13 beschrieben. Die Auswertung erfolgt durch Autoradiographie der Phosphorcellulose (P 81)-Traeger.

## Beispiel 19

Immunologischer Nachweis von HSA nach Protein-Blotting auf Nitrocellulose-Membranen unter proteinfreien Blockierungsbedingungen

Die Durchfuehrung des Verfahrens erfolgt, wie es im Beispiel 13 beschrieben worden ist. Jedoch wird als Kanamycin-beladener Traeger Phosphorcellulose (P 81, Whatman) eingesetzt.

Die Auswertung erfolgt durch Autoradiographie der Phosphorcellulose mittels Roentgenfilm (Exposition: 15 Minuten, Zimmertemperatur).

Beispiel 20

Immunologischer Nachweis von HSA nach Protein-Blotting auf Nitrocellulose-Membranen unter proteinfreien Blockierungsbedingungen (Kontaktverfahren mit Neomycin-beladener Phosphorcellulose

Die Durchfuehrung des Gesamtverfahrens erfolgt, wie es im Beispiel 13 beschrieben worden ist. Es wird jedoch als mit Antibiotikum-beladener Traeger mit Neomycin beladene Phosphorcellulose (Inkubation der Phosphorcellulose 30 Minuten mit einer Loesung aus 400 mg Neomycin in 60 ml Wasser und Waschen mit Wasser 30 Minuten) eingesetzt. Die Auswertung erfolgt durch Autoradiographie der Phosphorcellulose mittels Roentgenfilm (Exposition: 15 Minuten, Zimmertemperatur).

Beispiel 21

Immunologischer Nachweis von HSA nach Blotting von Proteinen auf Nitrocellulose unter proteinfreien Blockierungsbedingungen (Kontakt mit Phosphorcellulose, Kanamycin im Enzymreaktions-Puffer)

Die Durchfuehrung des Gesamtverfahrens erfolgt wie im Beispiel 13. Als Kontakttraeger wird jedoch Phosphorcellulose (P 81, Whatman) ohne vorherige Beladung mit Antibiotika verwendet.

Dem Enzympuffer wird Kanamycin mit einer Konzentration von 4 mg/ml zugesetzt.

Die Auswertung erfolgt durch Autoradiographie der Phosphorcellulose.

Beispiel 22

Wiederverwendung von Protein-Blots

Die Protein-Blots auf Nitrocellulose (Hybond C) werden nach der Durchfuehrung des Gesamtverfahrens, wie es im Beispiel 13 beschrieben worden ist (Runde 1), bei 70°C 2 mal 15 Minuten mit dem Elutionspuffer 3 % SDS, 0,1 M β-Mercaptoethanol in TBS gewaschen und damit der 1. Antikoerper einschliesslich der enzymmarkierten Sonde eluiert. Nach der Waschung wird mit TBS + 0,1 % Tween 20 erneut geblockt und das Verfahren, wie es im Beispiel 13 beschrieben worden ist, wiederholt. Die Kontakttraeger der 1. und der 2. Runde werden gemeinsam autoradiographiert. Es wird kein Intensitaetsverlust nach der Wiederverwendung der Blots festgestellt.

Beispiel 23

Immunologischer Nachweis von HSA nach Proteinblotting auf Nitrocellulose unter proteinfreien Blockierungsbedingungen (Kontakttraeger: mit Kanamycin beladene Carboxymethyl-Cellulose

Die Durchfuehrung des Gesamtverfahrens erfolgt, wie es im Beispiel 13 beschrieben worden ist. Als Kontakttraeger wird mit Kanamycin beladene Carboxymethyl-Cellulose (CM-Membranen, Renner GmbH) verwendet. Die Auswertung erfolgt durch Autoradiographie der CM-Membranen.

Beispiel 24

Immunologischer Nachweis von HSA nach Protein-Blotting auf Nitrocellulose unter proteinfreien Blokkungsbedingungen (Verwendung eines Kopplungsproduktes aus Protein A und NPT II mit einem Toluylendiisocyanat-2,4-Praepolymeren)

A) Herstellung der enzymmarkierten Sonde

a) Elektrophoretische Reinigung von NPT II aus E.coli-Lysaten

Das Zellpellet von 10 ml E.coli (NPT II ueberexprimierend) Zellkultur (pRK 89) wird in 1 ml Lysepuffer aufgenommen und beschallt. Zellreste werden bei 4°C abzentrifugiert. Das Zellysat wird auf ein praeparatives PAA/SDS-Gel aufgetragen und elektrophoretisch aufgetrennt. Das Gel wird nach Waschen mit Wasser in eine kalte Loesung von 0,25 M KCl gelegt, die sichtbar gewordene NPT II-Bande mit einem Skalpell herausgeschnitten und zerkleinert. Das Protein wird mittels Elutionspuffer innerhalb von 3 Stunden bei Zimmertemperatur eluiert. Die durch Elution erhaltenen Loesungen werden zentrifugiert und der Ueberstand mit dem vierfachen Volumen kalten Acetons versetzt und 3 Stunden bei -20°C stehengelassen. Danach wird bei 0°C zentrifugiert. Nach Waschen wird das Pellet in 200 µl Loesepuffer geloest und gegen 20 mM Natriumphosphatpuffer ueber Nacht dialysiert (4°C). Im Endvolumen von etwa 200 µl befinden sich ungefaehr 200 µg NPT II.

b) Kopplung

Zur Kopplung wird ein Praepolymeres aus 17,4 g frisch destilliertem Toluylendiisocyanat-2,4 und 30 g Polyethylenglykol 600 bei 50 - 60°C hergestellt. 100 mg dieses Praepolymeren werden in 500 mg Aceton geloest; nach vollstaendiger Loesung werden 300 mg dieser Loesung in 700 mg Wasser emulgiert.

800 µl des unter a) erhaltenen Lysates werden mit 150 µg Protein A in 150 µl Wasser in einem Eppendorf-Roehrchen vermischt. Zu dieser Loesung werden 200 µl der oben hergestellten Emulsion zugegeben und 1 Stunde unter Schuetteln umgesetzt. Nach einer Stunde werden erneut 100 µl frisch hergestellte Emulsion zugesetzt und noch eine Stunde umgesetzt. Danach wird mit einer Loesung von 200 µg Lysin in 150 µl Wasser 30 Minuten bei Zimmertemperatur gestoppt.

c) Nachweis

Die Durchfuehrung des Gesamtverfahrens erfolgt wie in Beispiel 13. Jedoch wird an Stelle des gentechnisch hergestellten Protein A-NPT II-Fusionsproteins 50 µl des oben hergestellten Kopplungsproduktes als enzymmarkierte Sonde verwendet.

Der Nachweis erfolgt wieder durch Autoradiographie.

Beispiel 25

Immunologischer Nachweis von HSA nach Tuepfeln auf CCA-Papier (Kontakttraeger nach DD-WP 254.012)

Eine Verduennungsreihe von HSA wird auf CCA-Papier (hergestellt nach EP 0 134.025) aufgetuepfelt und 30 Minuten an der Luft trocknen gelassen. Danach wird der Traeger in eine Loesung aus 10 % Sulfanilsaeure und 10 % Triethanolamin (pH = 7,5) eine Stunde bei Zimmertemperatur unter leichtem Schuetteln inkubiert. Der Block I erfolgt in TBS mit 0,5 % Gelatine und 30 Minuten in TBS mit 0,25 % Gelatine. Danach wird das Verfahren wie in Beispiel 14 durchgefuehrt.

Die Auswertung erfolgt durch Autoradiographie des Kontakttraegers nach DD-WP 254.012 und des CCA-Papiers. Auf beiden Traegern laesst sich durch $^{32}P$-$\gamma$-ATP markiertes Kanamycin nachweisen.

Beispiel 26

Immunologischer Nachweis von HSA nach Tuepfeln auf ein negativiertes Papier (Kontakttraeger Phosphorcellulose)

Ein negativierter Traeger wird aus CCA-Papier und Sulfanilsaeure nach DD-WP 237.841 hergestellt. Auf diesen Traeger wird eine Verduennungsreihe von HSA aufgetuepfelt und als Blockierungsmittel (Block I und II) eine Loesung von 10 % Sulfanilsaeure und 10 % Triethanolamin vom pH = 7,5 verwendet. Als mit Kanamycin-beladener Traeger wird mit Kanamycin beladene Phosphorcellulose (P 81, Whatman) verwendet. Der Nachweis erfolgt dann wie in Beispiel 13. Der Nachweis des HSA erfolgt durch Autoradiographie an beiden Traegern.

Beispiel 27

Immunologischer Nachweis von HSA nach Tuepfeln auf einen hydrophoben Traeger (Kontakttraeger nach DD-WP 254.012)

Ein hydrophober Traeger wird nach DD-WP durch Umsetzung des CCA-Papiers (nach EP 0 134.025) mit aequimolekularen Mengen Di-n-octylamin und Sulfanilsaeure als Natriumsalz hergestellt. Auf diesen Traeger wird eine HSA-Verduennungsreihe aufgetragen. Nach 30 Minuten bei Zimmertemperatur sind die Tropfen auf dem Traeger eingetrocknet, man laesst noch weitere 15 Minuten nachtrocknen. Die Blockierung erfolgt im Block I und II mit TBS plus Gelatine, wie es in Beispiel 14 beschrieben worden ist. Die Durchfuehrung des Verfahrens erfolgt ansonsten, wie es im Beispiel 13 beschrieben worden ist.

Der Nachweis erfolgt durch Autoradiographie auf beiden Traegern.

Beispiel 28

Immunologischer Nachweis von HSA nach Proteinblotting auf Nitrocellulose (Kontakttraeger
Phosphorcellulose) und Verstaerkung der Nachweisreaktion

Eine Verduennungsreihe wird auf Nitrocellulose
(Hybond C, Amersham) geblottet und anschliessend geblockt, wie es im Beispiel 13 beschrieben
worden ist. Nach der Inkubation mit dem ersten
Antikoerper wird zunaechst mit TBS + 0,1 % Tween 20 1 Stunde gewaschen, danach wieder geblockt (TBS mit 0,2 % Tween 20) und mit 20 µg
Protein A in 10 ml Loesung leicht geschuettelt.
Daran anschliessend wird erneut mit TBS + 0,1 %
Tween 20 gewaschen und erneut geblockt.

100 µl des vorgebildeten Komplexes aus HSA-
Antihumanserumalbumin-Protein-A-NPT II-Fusionsprotein (hergestellt aus 50 µl Lysat nach Beispiel 6,
50 µg HSA und 50 µl Anti-HSA-Immunglobulin
(Kaninchen), Inkubation bei 4°C ueber Nacht) werden dann zugesetzt und 1 Stunde inkubiert. Anschliessend wird mit TBS gewaschen und das Kontaktverfahren nach Beispiel 13 mit einem mit Kanamycin beladenen Traeger nach DD-WP 254.012
durchgefuehrt. Die Auswertung erfolgt durch Autoradiographie des Kontakttraegers (30 Minuten bei
Zimmertemperatur.

Beispiel 29

Immunologischer Nachweis von α-Fetoprotein nach
Tuepfeln auf Nitrocellulose (Kontakttraeger nach
DD-WP 254.012) -Verstaerkung durch mit Antikoerper vernetztes Protein A-NPT II-Fusionsprotein

Die ersten Schritte des Verfahrens erfolgen,
wie es im Beispiel 15 beschrieben worden ist.
Nach der Inkubation mit dem 1. Antikoerper
(monoklonal) wird mit TBS + 0,1 % Tween 20
gewaschen und mit TBS + 0,2 % Tween 20 geblockt. Danach werden 50 µl biologisch vernetztes
Protein A-NPT II-Fusionsprotein (vgl. Beispiel 11)
zugesetzt und 1 Stunde inkubiert.

Nach der Waschung mit TBS (1 Stunde) wird
weiter wie in Beispiel 15 verfahren. Die Auswertung
erfolgt durch Autoradiographie des Kontakttraegers
in 20 Minuten bei Zimmertemperatur.

Beispiel 30

Immunologischer Nachweis von HSA (Enzym-Reaktion in Loesung)

Eine Verduennungsreihe von HSA wird auf einzelne Rundfilter aus Nitrocellulose (Hybond C,
Amersham Int.) aufgetuepfelt (Durchmesser 0,5
cm²) und zunaechst wie in Beispiel 13 weiter bearbeitet. Nach dem Inkubieren mit Protein A-NPT II-
Fusionsprotein werden die Filter 1 Stunde in TBS
gewaschen und danach einzeln in Plastebehaelter
ueberfuehrt. Es werden jeweils 0,5 ml Enzympuffer,
1 mg Kanamycin und $^{32}$P-γ-ATP (10 Mio. cpm/ml,
spezifische Aktivitaet 5 000 Ci/mM) zugesetzt und
1 Stunde bei 37°C umgesetzt. Die Nitrocellulose-
Membranen werden entnommen, ein Rundfilter aus
Phosphorcellulose (P 81, 1 cm²) zugesetzt und 30
Minuten geschuettelt. Die Phosphorcellulose-Filter
werden entnommen, 1 Stunde mit Wasser gewaschen, getrocknet und im Szintillationszaehler gemessen. Die Auswertung erfolgt direkt ueber den
Messwert der Radioaktivitaet (Als Kontrolle wird ein
Nitrocellulose-Filter ohne HSA mitgefuehrt).

**Ansprüche**

1. Markierte molekulare Sonden aus einer molekularen Sonde und einem markierenden Enzym,
dadurch gekennzeichnet,
daß sie aus molekularen Sonden bestehen, die
direkt oder über einen Spacer an Transferasen
(E.C.2) oder Ligasen (E.C.6) gekoppelt sind.

2. Markierte molekulare Sonden nach Anspruch
1, dadurch gekennzeichnet, daß die molekularen
Sonden durch kovalente Bindung, über ionische
Gruppen und/oder durch elektrostatische Wechselwirkungen an die Enzymmoleküle gebunden sind.

3. Markierte molekulare Sonden nach Anspruch
1 oder 2, dadurch gekennzeichnet, daß das Enzym
eine Aminglucosid-Phosphotransferase ist.

4. Markierte molekulare Sonden nach einem
der Ansprüche 1 bis 3, dadurch gekennzeichnet,
daß die mokularen Sonden immunreaktive Sonden
sind.

5. Markierte molekulare Sonden nach Anspruch
4, dadurch gekennzeichnet, daß die immunreaktiven Sonden monoklonale oder polyklonale Antikörper, Antiseren, Proteine oder Peptide sind.

6. Markierte molekulare Sonden nach Anspruch
5, dadurch gekennzeichnet, daß das Protein Protein
A ist.

7. Markierte molekulare Sonden nach einem
der Ansprüche 1 bis 3, dadurch gekennzeichnet,
daß die molekularen Sonden natürlich vorkommende, chemisch hergestellte oder gentechnisch erzeugte Nucleinsäuren oder deren Derivate oder
Fragmente davon sind.

8. Markierte molekulare Sonden nach Anspruch 7, dadurch gekennzeichnet, daß die Nucleinsäuren einsträngige DNS oder Fragmente davon, doppelsträngige oder partiell doppelsträngige DNS oder Fragmente davon, Oligodesoxyribonucleotide, RNS oder Fragmente davon oder Oligoribonucleotide sind.

9. Markierte molekulare Sonden nach einem der Ansprüche 1 bis 6, dadurch gekennzeichnet, daß die molekularen Sonden Proteine mit spezifischer Affinität zu einem anderen Biomolekül sind.

10. Markierte molekulare Sonden nach einem der Ansprüche 6 bis 9, dadurch gekennzeichnet, daß die markierten molekularen Sonden Fusionsproteine aus einem Protein und dem Enzym sind.

11. Verfahren zur Herstellung der markierten molekularen Sonden nach einem der Ansprüche 1 bis 10, dadurch gekennzeichnet, daß an eine molekulare Sonde oder ein Gemisch molekularer Sonden direkt oder über einen Spacer eine Transferase (E.C.2) oder Ligase (E.C.6) gekoppelt wird.

12. Verfahren nach Anspruch 11, dadurch gekennzeichnet, daß die molekularen Sonden durch kovalente Bindung, über ionische Gruppen und/oder durch elektrostatische Wechselwirkungen direkt oder über einen Spacer an die Enzymmoleküle gebunden werden.

13. Verfahren nach Anspruch 11 oder 12, dadurch gekennzeichnet, daß an eine molekulare Sonde oder ein Gemisch molekularer Sonden eine Aminoglucosid-Phosphotransferase gekoppelt wird.

14. Verfahren nach einem der Ansprüche 11 bis 13, dadurch gekennzeichnet, daß als molekulare Sonden immunreaktive Sonden und insbesondere mono- oder polyklonale Antikörper, Antiseren, Proteine oder Peptide eingesetzt werden.

15. Verfahren nach einem der Ansprüche 11 bis 14, dadurch gekennzeichnet, daß als molekulare Sonden eine oder mehrere natürlich vorkommende, chemisch hergestellte oder gentechnisch erzeugte Nucleinsäuren oder deren Derivate oder Fragmente eingesetzt werden.

16. Verfahren nach Anspruch 15, dadurch gekennzeichnet, daß als Nucleinsäuren einsträngige DNS oder Fragmente davon, doppelsträngige oder partiell doppelsträngige DNS oder Fragmente davon, Oligodesoxyribonucleotide, RNS oder Fragmente davon oder Oligoribonucleotide eingesetzt werden.

17. Verfahren nach einem der Ansprüche 11 bis 16 und insbesondere Anspruch 10, dadurch gekennzeichnet, daß Fusionsproteine, die aus einem Protein und einer Aminoglucosid-Phosphotransferase bestehen, insbesondere gentechnisch hergestellt werden.

18. Verfahren zum Nachweis und zur Bestimmung von Biomolekülen mittels markierter molekularer Sonden, dadurch gekennzeichnet, daß zur Erkennung in Lösung befindlicher, in einem Gel enthaltener oder an eine feste Phase I gebundener Biomoleküle eine enzymmarkierte molekulare Sonde nach einem der Ansprüche 1 bis 10 zugegeben wird, das an die molekulare Sonde gekoppelte oder davon abgespaltene Enzym an einem Substrat, ggfs. in Anwesenheit eines Cosubstrates, zur Reaktion gebracht und das umgesetzte Substrat, insbesondere an einer festen Phase II oder III, nachgewiesen oder quantitativ bestimmt wird.

19. Verfahren nach Anspruch 18, dadurch gekennzeichnet, daß der Nachweis der Biomoleküle in folgenden Schritten durchgeführt wird:

a) Erkennungsschritt, in dem die durch ein gekoppeltes Enzym markierte molekulare Sonde an das in Lösung befindliche, in einem Gel enthaltene oder an eine feste Phase I gebundene Biomolekül mit dafür spezifischer Affinität angelagert wird;

b) Waschschritt, in dem überschüssige markierte molekulare Sonde ausgewaschen und/oder abgetrennt wird;

c) Enzymreaktionsschritt, in dem das Substrat, erforderlichenfalls in Anwesenheit eines Cosubstrates, durch das Enzym umgesetzt wird;

d) Trennschritt, in dem ggfs. nicht umgesetztes Cosubstrat und ggfs. Substrat ausgewaschen oder abgetrennt werden;

e) Auswertungsschritt, in dem das umgesetzte Substrat an einer festen Phase II oder III gemessen oder durch geeignete Nachweisverfahren nachgewiesen wird.

20. Verfahren nach Anspruch 19, dadurch gekennzeichnet, daß
- im Erkennungsschritt a) Biomoleküle in Gelen oder anderen Trenn- und Trägermaterialien aufgetrennt werden und die die Biomoleküle enthaltenden Trenn- und Trägermaterialien mit einer die enzymgekoppelte molekulare Sonde enthaltenden Lösung in Kontakt gebracht werden, wobei Biomolekül und markierte molekulare Sonde zu einem Komplex aus Biomolekül und enzymgekoppelter Sonde im Gel oder Trenn- und Trägermaterial umgesetzt werden;
- im Waschschritt b) überschüssige markierte molekulare Sonde aus dem Gel oder Trenn- und Trägermaterial ausgewaschen wird;
- im Enzymreaktionsschritt c) das Gel oder Trenn- und Trägermaterial mit einem zweiten Gel oder Trenn- und Trägermaterial, das das Substrat und ggfs. das Cobsubstrat enthält, in Kontakt gebracht und das Substrat umgesetzt wird;
- im Trennschritt d) das zweite Gel oder Trenn- und Trägermaterial mit dem umgesetzten Substrat mit einer festen Phase III in Kontakt gebracht und das umgesetzte Substrat an ihr gebunden wird, und nach Waschschritten
- im Auswertungsschritt e) das umgesetzte Substrat auf der festen Phase III durch Messung der Radioaktivität oder durch Autoradiographie oder ein anderes Verfahren nachgewiesen wird.

21. Verfahren nach Anspruch 19 oder 20, dadurch gekennzeichnet, daß nach den Schritten a) und b) das Gel oder Trenn- und Trägermaterial, das den Komplex aus Biomolekül und enzymmarkierter molekularer Sonde enthält, mit einer festen Phase II, an die das Substrat gebunden ist, in Gegenwart eines Cosubstrates in Kontakt gebracht und das enzymatisch umgesetzte Substrat auf der festen Phase II nachgewiesen werden.

22. Verfahren nach einem der Ansprüche 18 bis 21, dadurch gekennzeichnet, daß als feste Phase I, an die das Biomolekül˙ gebunden wird, ein kovalent, ionisch oder über elektrostatische Wechselwirkungen bindender Träger eingesetzt wird.

23. Verfahren nach einem der Ansprüche 18 bis 22, dadurch gekennzeichnet, daß als feste Phase II ein als Substrat kovalent, ionisch oder über elektrostatische Wechselwirkungen bindender Träger eingesetzt wird.

24. Verfahren nach einem der Ansprüche 18 bis 23, dadurch gekennzeichnet, daß als feste Phase III ein das Substrat und/oder das enzymatisch umgesetzte Substrat selektiv kovalent, ionisch oder über elektrostatische Wechselwirkungen bindendes Gel, Trenn- oder Trägermaterial eingesetzt wird.

25. Verfahren nach einem der Ansprüche 22 bis 24, dadurch gekennzeichnet, daß als Träger Nitrocellulose, Polyvinylidendifluorid, Polyamid, geladenes Polyamid, Diazobenzyloxymethylcellulose, Diethylaminoethylcellulose, durch Cyanurchlorid aktivierte Cellulose, aktiviertes Polystyrol, chemisch aktiviertes Glas, Phosphorcellulose, Carboxymethylcellulose oder durch Cyanurchlorid und eine Verbindung, die eine Aminogruppe und eine Carbonsäure-, Phosphorsäure- oder Sulfonsäuregruppe aufweist, modifizierte Cellulose eingesetzt werden.